# EUROPEAN PATENT APPLICATION

(11) **EP 3 082 098 A1**
(43) Date of publication of application: **19.10.2016**
(21) Application number: 14868986.2
(22) Date of filing: 21.05.2014
(51) Int. Cl.: G06Q 50/22

(54) **VACCINE SCHEDULING DEVICE, VACCINE SCHEDULING PROGRAM, AND COMPUTER-READABLE RECORDING MEDIUM STORING SUCH PROGRAM**

(30) Priority: 12.12.2013 JP 2013257088
(71) Applicant: The Institute of Medical Science And Research, Tokyo 150-0022 (JP)
(72) Inventor: KATOU, Itaru, Tokyo 107-0052 (JP)
(74) Representative: Green, Mark Charles
(86) International application number: PCT/JP2014/002659
(87) International publication number: WO 2015/087464

(57) **Abstract**

Provided are a vaccine scheduling device and vaccine scheduling program making it possible to put together a schedule for preventive vaccination in a manner more suitable for a preventive vaccination subject, as well as a computer-readable recording medium storing this program.

A schedule creation unit (13) has a provisional schedule creation unit (13a) and a final schedule creation unit (13b). The final schedule creation unit (13b) reads information on a prohibition period corresponding to a past disease indicated by a designated person's clinical history data received by a clinical history data receiving unit (14), sets a prohibition period starting from a fixed date indicated by the clinical history data and, if there are scheduled administration dates in a provisional schedule created by the provisional schedule creation unit (13a) in the time period from the current point in time until a final date of the prohibition period, creates a final schedule in accordance with the number of days of difference between the earliest one of these scheduled administration dates and a release date when the prohibition period is released.

## Description

### [Technical Field]

The present invention relates to a vaccine scheduling device, a vaccine scheduling program and a computer-readable recording medium storing this program.

### [Background Art]

Heretofore, for example, the Japan Pediatric Society has proposed a preventive vaccination schedule which is recommended by this society. Vaccines which children should receive and vaccination periods which are recommended of these vaccines respectively are described in the schedule.

Generally speaking, with respect to preventive vaccination for children, guardians decide when and what vaccines are to be inoculated based on the doctor's instructions, periods recommended by local governments and the like. However, while there are a number of vaccines to be inoculated, it is not easy to create a preventive vaccination schedule by taking into consideration the vaccination interval period between one vaccine and another vaccine, simultaneous vaccination and so forth.

Accordingly, a system for creating preventive vaccination schedules has been proposed (for example, refer to Patent Document 1). This system automatically creates a schedule for an individual, and therefore it is possible to receive preventive vaccination in an appropriate manner without requiring a preventive vaccination subject or the guardian thereof to create a preventive vaccination schedule which is not easy.

### [CITATION LIST]

### [PATENT LITERATURES]

[PATENT LITERATURE 1] Japanese Patent Published Application No. 2012-59155

### [Summary of Invention]

### [Technical Problem]

However, the preventive vaccination schedule creation system described in Patent Document 1 does not perform scheduling based on the state of a preventive vaccination subject, and thereby still has room for improvement. For example, in the case of a child catching the measles, there is provided a period in which preventive vaccination cannot be carried out after the child is cured of the measles. However, the preventive vaccination schedule creation system described in Patent Document 1 takes into consideration the interval period (four weeks for live vaccine, one week for inactivated vaccine) after vaccination of a vaccine before vaccination of another vaccine, but does not take into consideration the current or past state of a preventive vaccination subject, so that it is insufficient to create a schedule which is more suitable for the preventive vaccination subject.

It is therefore an object of the present invention to provide a vaccine scheduling device, a vaccine scheduling program and a computer-readable recording medium storing this program capable of creating a preventive vaccination schedule which is more suitable for a preventive vaccination subject.

### [Means to solve the Problems]

A vaccine scheduling device of the present invention is structured to create a schedule of preventive vaccination, and comprises: a first storing means which stores information about a plurality of vaccines to be administered, and stores, in association with the information about the plurality of vaccines respectively, the information about priority levels of the vaccines, and information about the interval period to be inserted when administering, after each vaccine is administered, another vaccine which is different from the each vaccine; a second storing means which stores information about vaccines which have been administered to a plurality of preventive vaccination subjects respectively; a designation means structured to designate one of the plurality of preventive vaccination subjects as a designated person; a determination means structured to select vaccines one by one in the descending order of the priority levels based on the information about the priority levels stored in association with the information of the vaccines stored in the first storing means, and successively determine whether or not the selected vaccine has been administered to the designated person based on the information stored in the second storing means; a schedule creation means structured to create a schedule with the vaccines successively determined by the determination means as not having administered to the designated person; a third storing means which stores, for each of a plurality of diseases, information about a prohibition period in which administration of the each vaccine is prohibited from a fixed date of the each disease; and a clinical history data receiving means which receives clinical history data which includes past diseases of the designated person and fixed dates thereof, the schedule creation means comprising: a provisional schedule creation means which successively selects, one by one in the descending order of the priority levels, the vaccines which are successively determined by the determination means as not having administered to the designated person, sets a candidate administration date of the selected vaccine, resets the candidate administration date to the earliest date after the interval period of a vaccine which is administered just before the selected vaccine, and determines the reset candidate administration date as the scheduled administration date of the selected vaccine; a final schedule creation means which reads, from a third storing means, the prohibition period corresponding to a past disease of the clinical history data of the designated person received by the clinical history data receiving means, sets the prohibition period from the fixed date of the past disease of the clinical history data, and in the case where the scheduled administration date of a vaccine(s) is between the present time and the last day of the prohibition period in accordance with a provisional schedule created by the provisional schedule creation means, creates a final schedule by shifting to the future, in accordance with the differential number of days between the earliest scheduled administration date of the vaccine(s) and the release day when the prohibition period ends, the scheduled administration dates of vaccines to be administered after the fixed date of the past disease of the clinical history data.

Also, in the vaccine scheduling device of the present invention, the final schedule creation means may create the final schedule by shifting the scheduled administration date of the vaccines to be administered after the fixed dates of the clinical history data to the future by the differential date.

Furthermore, in the vaccine scheduling device of the present invention, it is preferred that the final schedule creation means creates the final schedule by shifting the scheduled administration date of the vaccines to be administered after the fixed dates of the clinical history data to the future by the differential date, and thereafter calculating the number of gap days of each of all the vaccines having been shifted to the future, and shifts to the past the scheduled administration dates of all the vaccines having been shifted to the future, by the number of gap days in a range not exceeding the differential date.

Still further, in the vaccine scheduling device of the present invention, it is preferred that, with respect to each of the vaccines to be administered after the fixed dates of the clinical history data, the final schedule creation means calculates the number of days by subtracting the number of gap days from the differential date, and shifts the scheduled administration date to the future by the calculated number of days if this calculated number of days is positive, to create the final schedule.

Still further, in the vaccine scheduling device of the present invention, it is preferred that the provisional schedule creation means sets a candidate administration date of the selected vaccine, resets the candidate administration date to the earliest date after the interval period of a vaccine which is administered just before the selected vaccine, and determines the reset date as the scheduled administration date of the vaccine if the interval period of the selected vaccine is secured between the reset date and the scheduled administration date just after this reset date.

Also, in the vaccine scheduling device of the present invention, it is preferred that the clinical history data receiving means further receives the clinical history data of at least either of the family of the designated person and a group to which the designated person belongs, wherein the further received clinical history data includes information about a past disease, the development confirmation date of the past disease, and the fixed date of the past disease, wherein the third storing means stores, with respect to each of a plurality of diseases, information about a prohibition period in which vaccination is prohibited after the fixed dates of the each disease, and information about the maximum value of an incubation period, and wherein the final schedule creation means reads the information about the prohibition period and the information about the maximum incubation period corresponding to the past diseases contained in the further received clinical history data from the third storage means, calculates an infection development period by subtracting the number of days, which elapsed from the development confirmation date of a past disease indicated by the further received clinical history data to the current date, from the maximum incubation period of this disease, sets an added period by adding the prohibition period of the disease to an infection development period, and creates a final schedule by, in the case where the scheduled administration date of a vaccine(s) described in the provisional schedule created by the provisional schedule creation means falls in the period from the current time to the last day of the added period, shifting the scheduled administration date of a vaccine(s) to be administered after the current time to the future in accordance with the differential number of days between the release day when the added period ends and the earliest scheduled administration date of the vaccine(s) falling in the period.

Furthermore, in the vaccine scheduling device of the present invention, the final schedule may be created by shifting the scheduled administration date of the vaccines to be administered after the fixed dates of the further received clinical history data to the future by the added period.

Still further, in the vaccine scheduling device of the present invention, it is preferred that the final schedule creation means shifts the scheduled administration date of the vaccines to be administered after the fixed dates of the further received clinical history data to the future by the added period, thereafter calculates the number of gap days of each of all the vaccines having been shifted to the future, and shifts to the past the scheduled administration dates of all the vaccines having been shifted to the future, by the number of gap days in a range not exceeding the differential date, to create the final schedule.

Still further, in the vaccine scheduling device of the present invention, it is preferred that, with respect to each of the vaccines to be administered after the development confirmation date of the further received clinical history data, the final schedule creation means calculates the number of days by subtracting the number of gap days from the differential date, and shifts the scheduled administration date to the future by the calculated number of days if this calculated number of days is positive, to create the final schedule.

Still further, in the vaccine scheduling device of the present invention, it is preferred that a fourth storing means is further provided for storing information about whether or not an adverse effect occurs after administering a vaccine to the designated person, and that the schedule creation means is structured to schedule, of the vaccines successively determined by the determination means as not having administered to the designated person, a vaccine which is not of the same type as a vaccine with which an adverse effect occurs.

Still further, in the vaccine scheduling device of the present invention, it is preferred that a fifth storing means is further provided for storing information about a vaccine which is administered to the designated person in the past and requires revaccination, and that the schedule creation means is structured to schedule the vaccines successively determined by the determination means as not having administered to the designated person, and the vaccine requiring revaccination stored in the fifth storing means.

Still further, in the vaccine scheduling device of the present invention, it is preferred to further provide a calculation means which collects final schedules for a plurality of persons created by the final schedule creation means, and calculates the types and numbers of vaccines to be administered in a predetermined period; and a comparison order means which compares the types and numbers of vaccines to be administered in the predetermined period calculated by the calculation means with the types and numbers of vaccines in stock, and ordering shortage amounts.

Also, a vaccine scheduling program of the present invention causes a vaccine scheduling device structured to create a schedule of preventive vaccination and comprising a first storing means which stores information about a plurality of vaccines to be administered, and stores, in association with the information about the plurality of vaccines respectively, the information about priority levels of the vaccines, and information about the interval period to be inserted when administering, after each vaccine is administered, another vaccine which is different from the each vaccine; a second storing means which stores information about vaccines which have been administered to a plurality of preventive vaccination subjects respectively; and a third storing means which stores, for each of a plurality of diseases, information about a prohibition period in which administration of the each vaccine is prohibited from a fixed date of the each disease, to perform: a designation step of designating one of the plurality of preventive vaccination subjects as a designated person; a determination step of selecting vaccines one by one in the descending order of the priority levels based on the information about the priority levels stored in association with the information of the vaccines stored in the first storing means, and successively determining whether or not the selected vaccine has been administered to the designated person based on the information stored in the second storing means; a schedule creation step of creating a schedule with the vaccines successively determined by the determination step as not having administered to the designated person; and a clinical history data receiving step of receiving clinical history data which includes past diseases of the designated person and fixed dates thereof, the schedule creating step comprising: a provisional schedule creation step of successively selecting, one by one in the descending order of the priority levels, the vaccines which are successively determined in the determination step as not having administered to the designated person, setting a candidate administration date of the selected vaccine, resetting the candidate administration date to the earliest date after the interval period of a vaccine which is administered just before the selected vaccine, and determining the reset candidate administration date as the scheduled administration date of the selected vaccine; and a final schedule creation step of reading, from a third storing means, the prohibition period corresponding to a past disease of the clinical history data of the designated person received by the clinical history data receiving step, setting the prohibition period from the fixed date of the past disease of the clinical history data, and in the case where the scheduled administration date of a vaccine(s) is between the present time and the last day of the prohibition period in accordance with a provisional schedule created by the provisional schedule creation step, creating a final schedule by shifting to the future, in accordance with the differential number of days between the earliest scheduled administration date of the vaccine(s) and the release day when the prohibition period ends, the scheduled administration dates of vaccines to be administered after the fixed date of the past disease of the clinical history data.

A computer-readable recording medium storing the vaccine scheduling program of the present invention is a medium storing the vaccine scheduling program as recited in the above.

Incidentally, the preventive vaccination subject referred to in the above is a person who is registered in advance, and to be scheduled to administer vaccines by the vaccine scheduling device and the program, and is for example a child who is registered in advance by a guardian.

### [Advantageous Effects of Invention]

In accordance with these present inventions, a final schedule is created by shifting the scheduled administration date of the vaccines to be administered after the fixed dates of the clinical history data to the future. Because of this, it is possible to create a final schedule by setting a prohibition period in appropriate manner for a designated person who has the measles, the rubella, the chicken pox or the like. Accordingly, it is possible to create a schedule taking into consideration the prohibition period of a vaccine based on the clinical history, and create a preventive vaccination schedule in an appropriate manner for a preventive vaccination subject.

### [Brief Description of the Drawings]

[Fig. 1] Fig. 1 is an external view for showing a vaccine scheduling device in accordance with an embodiment of the present invention.
[Fig. 2] Fig. 2 is a hardware configuration view for showing the vaccine scheduling device in accordance with a first embodiment.
[Fig. 3] Fig. 3 is a first view showing an example of the display screen of the vaccine scheduling device in accordance with the first embodiment.
[Fig. 4] Fig. 4 is a second view showing another example of the display screen of the vaccine scheduling device in accordance with the first embodiment.
[Fig. 5] Fig. 5 is a view to show the stored contents of an HDD and a USB.
[Fig. 6] Fig. 6 is a view showing the stored contents stored in a first storage area.
[Fig. 7] Fig. 7 is a view showing the stored contents stored in a second storage area.
[Fig. 8] Fig. 8 is a view showing the stored contents stored in a third storage area.
[Fig. 9] Fig. 9 is a view for showing the functional configuration of the vaccine scheduling device in accordance with the first embodiment.
[Fig. 10] Fig. 10 is a third view showing an example of the display screen of the vaccine scheduling device in accordance with the first embodiment.
[Fig. 11] Fig. 11 is a fourth view for showing an example of the display screen of the vaccine scheduling device in accordance with the first embodiment.
[Fig. 12] Fig. 12 is a view for showing an example of the clinical history data of a designated person.
[Fig. 13] Fig. 13 is a flow chart for showing a schedule creation method by the vaccine scheduling device in accordance with the first embodiment.
[Fig. 14] Fig. 14 is a flow chart for showing the schedule subject determination process (S1) shown in Fig. 13 in detail.
[Fig. 15] Fig. 15 is a flow chart for showing the provisional schedule creation process (S2) shown in Fig. 13 in detail.
[Fig. 16] Fig. 16 is a first flow chart showing the schedule determination process (S29) shown in Fig. 15 in detail.
[Fig. 17] Fig. 17 is a second flow chart showing the schedule determination process (S29) shown in Fig. 15 in detail.
[Fig. 18] Fig. 18 is a third flow chart showing the schedule determination process (S29) shown in Fig. 15 in detail.
[Fig. 19] Fig. 19 is a fourth flow chart showing the schedule determination process (S29) shown in Fig. 15 in detail.
[Fig. 20] Fig. 20 is a flow chart for showing the final schedule creation process (S3) shown in Fig. 13 in detail.
[Fig. 21] Fig. 21 is a flow chart for showing the process of the vaccine scheduling device in accordance with the first embodiment and showing the process of ordering vaccines.
[Fig. 22] Fig. 22 is a view for showing the stored contents of the HDD and the USB in accordance with the second embodiment.
[Fig. 23] Fig. 23 is a view for showing the stored contents stored in the third storage area in accordance with the second embodiment.
[Fig. 24] Fig. 24 is a flow chart for showing the final schedule creation process (S3) in accordance with the second embodiment in detail.
[Fig. 25] Fig. 25 is a view for showing the stored contents of the second storage area in accordance with the third embodiment.
[Fig. 26] Fig. 26 is a flow chart for showing the provisional schedule creation process (S2) in accordance with the third embodiment in detail.
[Fig. 27] Fig. 27 is a view for showing the stored contents of the second storage area in accordance with the fourth embodiment.
[Fig. 28] Fig. 28 is a flow chart for showing the provisional schedule creation process (S2) in accordance with the fourth embodiment in detail.
[Fig. 29] Fig. 29 is a flow chart for showing the public expense process to be performed in step ST1 of Fig. 17 in detail.

### [DESCRIPTION OF EMBODIMENTS]

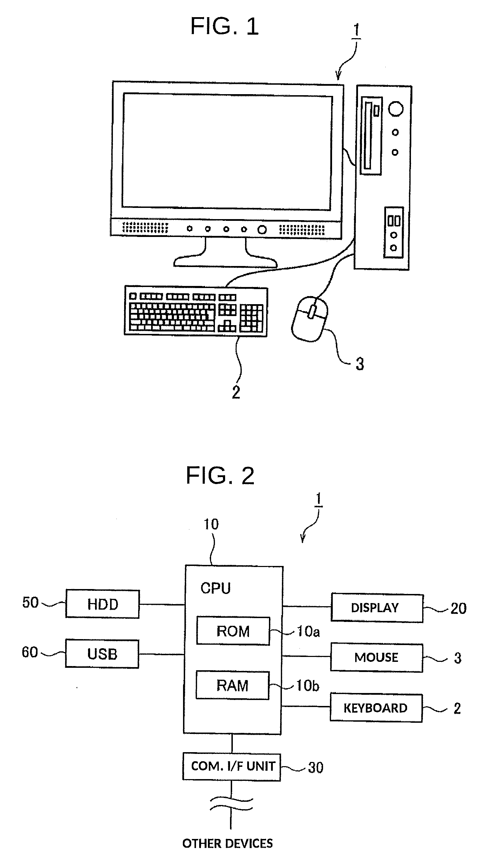

In what follows, a preferred embodiment of the present invention will be explained in accordance with the accompanying drawings. However, the present invention is not limited to this embodiment. Fig. 1 is an external view for showing a vaccine scheduling device in accordance with an embodiment of the present invention. The vaccine scheduling device 1 shown in Fig. 1 is implemented, for example, as one functionality of a personal computer for creating a schedule of preventive vaccinations to be received by a preventive vaccination subject. This vaccine scheduling device 1 is provided with an input means such as a keyboard 2 and a mouse 3 and creates a schedule through operation of the input means.

Incidentally, while the following example is explained in the case where the vaccine scheduling device 1 is used in a medical institution such as a hospital, a clinic or a healthcare center, the vaccine scheduling device 1 is used not only in a medical institution, but can be used also at home or in a local government. Furthermore, the vaccine scheduling device 1 can be used in any other place if applicable.

Fig. 2 is a hardware configuration view for showing the vaccine scheduling device 1 in accordance with a first embodiment. As shown in Fig. 2, the vaccine scheduling device 1 is provided with a Central Processing Unit (CPU) (designation means, determination means, schedule creation means, clinical history data reception means, calculation means, and comparison order means) 10, a display 20, a communication Interface unit 30, an Hard Disk Drive (HDD) (first to third storage meanss) 50, and a Universal Serial Bus (USB) (first to third storage means) 60.

The CPU 10 serves to control the entirety of the vaccine scheduling device 1 in accordance with the first embodiment, and is provided with a ROM 10a and a RAM 10b as illustrated in Fig. 2. The ROM 10a is a read-only memory in which is stored a vaccine scheduling program for implementing the vaccine scheduling device 1. The RAM 10b is a read-write memory storing various data and having areas which are required for operational works of the CPU 10.

The display 20 serves to display an input image for manipulation with the keyboard 2 and the mouse 3, display a schedule which is created by the vaccine scheduling device 1. The communication Interface unit 30 is an interface for communication with other devices.

The HDD 50 and the USB 60 are auxiliary storage devices connected to the personal computer. These HDD 50 and USB 60 may store a vaccine scheduling program for implementing the vaccine scheduling device 1. Namely, the CPU 10 may realize the functions of the vaccine scheduling device 1 of the first embodiment in accordance with the program stored in the HDD 50 and USB 60.

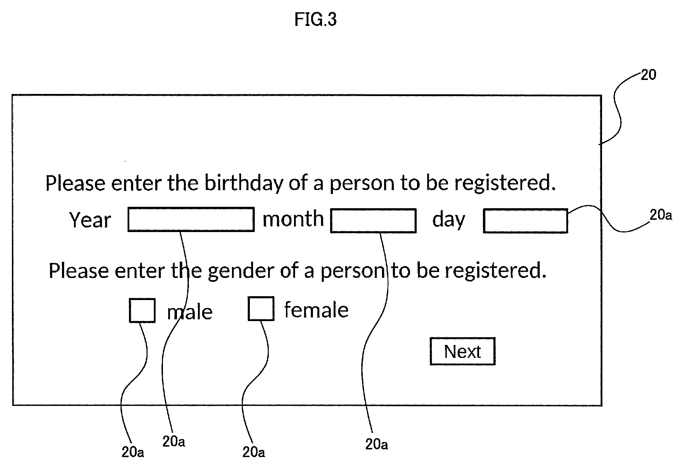

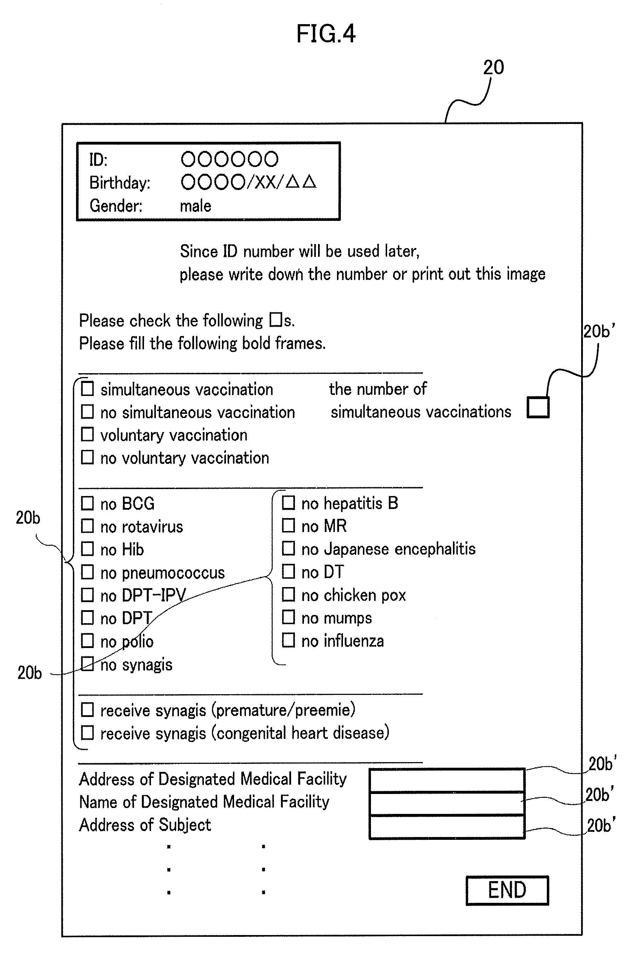

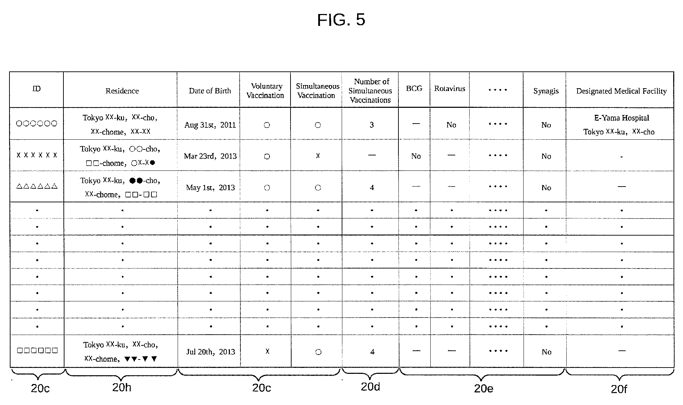

Also, as illustrated in Fig. 5, the HDD 50 and the USB 60 store basic information of users (preventive vaccination subjects) who are registered in advance. Next, in advance of the basic information, the screen shown in Fig. 3 and Fig. 4 will be explained. Fig. 3 is a first view showing an example of the display screen of the vaccine scheduling device 1 in accordance with the first embodiment. For example, the initial screen shown in Fig. 3 is displayed in the display 20 of the vaccine scheduling device 1. In this screen, input boxes 20a are displayed for entering a date of birth and a gender. From this screen as displayed, a user can proceed to the screen shown in Fig. 4 by entering a date of birth and a gender in the input boxes 20a with the input means and selecting "Next".

Fig. 4 is a second view showing another example of the display screen of the vaccine scheduling device 1 in accordance with the first embodiment. After entering a date of birth and a gender in the input boxes 20a and selecting "Next", the user is given an ID number (number for identification) as illustrated in Fig. 4. Also, a plurality of check boxes 20b and bold input boxes 20b' are displayed on the display 20 as illustrated in Fig. 4. The check boxes 20b are used to designate their contents by checking them with the input means. For example, if the check box 20b designating no simultaneous vaccination is checked, simultaneous vaccination is avoided of the user in creation of a preventive vaccination schedule. Simultaneous vaccination will be described later. On the other hand, if the check box 20b designating simultaneous vaccination is checked, simultaneous vaccination is allowed of the user in creation of a preventive vaccination schedule, and furthermore it becomes possible to enter a numeral in a bold input box 20b' indicative of the upper limit number of simultaneous vaccinations. In this case, if the user enters "3" in this bold input box 20b', three vaccines can be simultaneously administered. Also, if the check box 20b designating voluntary vaccination is checked, a preventive vaccination schedule is created for the user to carry out voluntary vaccinations for which no public expense is paid. On the other hand, if the check box 20b designating no voluntary vaccination is checked, a preventive vaccination schedule is created for the user not to carry out vaccinations at his own expense.

Furthermore, if the check box 20b designating no BCG is checked, a preventive vaccination schedule is created for the user not to receive a BCG vaccine. The other items such as rotavirus and Hib are treated in the same manner.

In addition to this, this device 1 can be used to input the address of the designated medical facility and the name of the designated medical facility through the bold input boxes 20b' respectively. In the case where the user desires to receive vaccination at the medical facility of a so-called family doctor, these input boxes are filled. Furthermore, the user can enter information in the bold input boxes 20b' for the residence of the user. This information about the residence is used for processing the subsidies of vaccination which are different depending upon local governments to be described below.

Still further, when the address of the designated medical facility and the name of the designated medical facility are entered, the characteristics of this medical facility may be used for overwriting with data stored in advance or through the communication Interface unit 30. For example, there are policies depending upon doctors in line with their beliefs such that simultaneous vaccination up to four vaccines is recommended, that Hib vaccination must be carried out and so on. When the address of the designated medical facility and the name of the designated medical facility are entered, the vaccine scheduling device 1 can reflect doctor's policies by forcibly overwriting input contents, whatever the user has input, with the policies.

Incidentally, DPT-IPV (Diphtheria, Pertussis, Tetanus and Inactivated Polio Vaccine) vaccines are substantially DPT (Diphtheria, Pertussis and Tetanus) vaccines plus poliovirus vaccine. For this reason, when none of DPT-IPV, DPT and poliovirus check boxes 20b is checked, it is preferred to automatically check either DPT-IPV check box or DPT and poliovirus check boxes to indicate no need for vaccination. Furthermore, since a doctor often has a policy also with respect to DPT-IPV, DPT and poliovirus, when the address of the designated medical facility and the name of the designated medical facility are entered, it is preferred that either vaccination is checked to indicate no need for vaccination in accordance with the doctor's policy.

After inputting the above information, "END" is pressed to complete registration. When registration is completed, the following stored contents are built in the vaccine scheduling device 1.

Fig. 5 is a view to show the stored contents of the HDD 50 and the USB 60. As illustrated in Fig. 5, for example, information 20c about the date of birth, whether to carry out arbitrary vaccinations and whether to carry out simultaneous vaccinations, information 20d about the upper limit number of simultaneous vaccinations, information 20e about whether not to administer vaccines respectively, information 20f about a designated medical facility and information 20h about a residence are stored in association with an ID number □□□□□□. Meanwhile, in Fig. 5, the data described in the lines above the ID number □□□□□□ is information of users (preventive vaccination subjects) who have been already registered.

Also, the HDD 50 and the USB 60 store the respective individual information in first to third storage areas in addition to the above basic information. First, the first storage area (first storage means) is used to store information about a plurality of vaccines to be inoculated (i.e., information about types of the vaccines which is hereinafter referred to as information of vaccine names), and store, in association with a plurality of vaccine names respectively, information about the priority information of the vaccines, and information about the interval period to be inserted when administering, after each vaccine is administered, another vaccine which is different from the each vaccine.

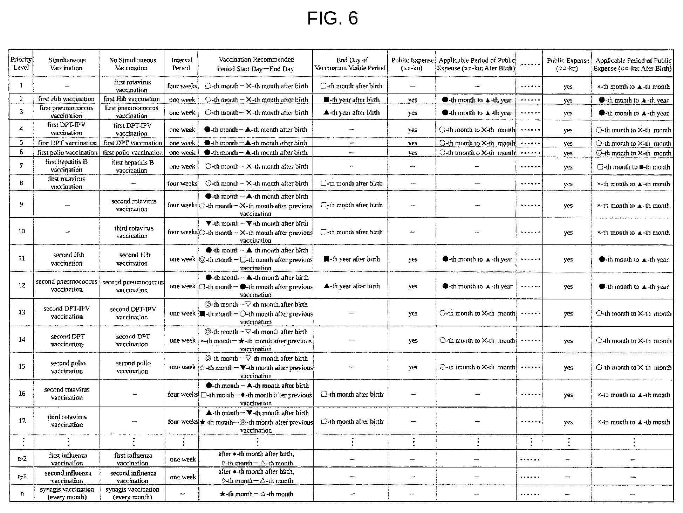

Fig. 6 is a view showing stored contents which are stored in the first storage area. As shown in Fig. 6, the names of vaccines to be inoculated are stored in the first storage area. These vaccine names are given priority levels respectively. These priority levels correspond to the order of receiving vaccination, and are determined in advance to set up appropriate vaccination timings in relation to the birth dates of preventive vaccination subjects respectively. For example, Hib, pneumococcus and the like have a high possibility of being developed soon after birth so that a vaccine has to be inoculated soon after birth. Since the disease may have already been developed even if vaccinated a certain time period after birth, the priority level is set to be high.

Furthermore, the first storage area is used to store the intervall periods in correspondence with vaccine names respectively. The interval period is a period to be inserted after a vaccine is administered, for receiving another vaccine which is different from the previous vaccine. For example, when a first Hib vaccination is carried out, it is forbidden just thereafter to carry out another vaccination such as a first pneumococcus vaccination, a first poliovirus vaccination, a second Hib vaccination. Unless one week has elapsed after the date of a first Hib vaccination, it is forbidden to carry out another vaccination such as a first pneumococcus vaccination, a first poliovirus vaccination, a second Hib vaccination. The first storage area is used to store such interval periods in association with vaccine names respectively.

In addition, in the first storage area, priority levels differ between the case where simultaneous vaccination is applicable and the case where simultaneous vaccination is not applicable. The simultaneous vaccination is such that a plurality of vaccines are administered at the same time. In Europe and America, ten or more vaccines can successively be administered (at the same day). Also in Japan, simultaneous vaccination is often recommended in infant's cases. Since a plurality of vaccines are administered in the case of simultaneous vaccination, it is no longer needed to consider the interval periods with respect to the plurality of vaccines. In other words, the interval period is a period to be inserted when another vaccine will be administered later. If a plurality of vaccines are successively administered in the same day, the interval period need not be considered with respect to these vaccines. Accordingly, in the case of no simultaneous vaccination where it is desired to carry out Hib, pneumococcus and poliovirus vaccinations in this order, the pneumococcus vaccine has to be administered one week after administering the Hib vaccine, and the poliovirus vaccine has to be administered further one week thereafter, so that two weeks are required in total as the interval period. Contrary to this, in the case of simultaneous vaccination, Hib, pneumococcus and poliovirus vaccinations can be carried out in the same day so that zero day is required as the interval period.

Also, the first storage area is used to store the start and end days of the vaccination recommended periods and the end days of the vaccination viable periods in correspondence with vaccine names respectively. For example, it is recommended to carry out the first vaccination of the Hib vaccine two months after birth. Because of this, with respect to the first vaccination of the Hib vaccine, the start day of the vaccination recommended period is set to the start day of two months after birth, and the end day is set to the day just before three months after birth. Likewise, the start and end days of the vaccination viable periods are stored in correspondence with vaccines respectively. Furthermore, the vaccination of the Hib vaccine is considered viable before four years after birth. Because of this, the end day of the vaccination viable period of the Hib vaccine is the day just before five years after birth. On the other hand, with respect to the second or later vaccination of a vaccine requiring plural times of vaccination such as the second vaccination of the rotavirus vaccine, a vaccination recommended period is set in relation to the previous administration date as a reference date, in addition to the vaccination recommended period in relation to the date of birth. Namely, using the first administration date of the rotavirus vaccine as a reference date, a vaccination recommended period is set, for example, from o-th month to ×-th months after the reference date. This is because unless the subsequent vaccination of the same vaccine is carried out within an appropriate period after the previous vaccination, it becomes difficult to produce the antibody in the human body.

Furthermore, the first storage area is used to store information about whether a public expense is paid or not and the applicable period of the public expense in correspondence with vaccine names. The information about whether a public expense is paid or not and the applicable period of the public expense is stored at each local government. This is because this information differs among local governments. Specifically, the first storage area is used to store the information about whether a public expense is paid or not (i. e., eligibility for public expense), and the information about the applicable period of the public expense of an eligible vaccine (i. e., the period in which the public expense is paid).

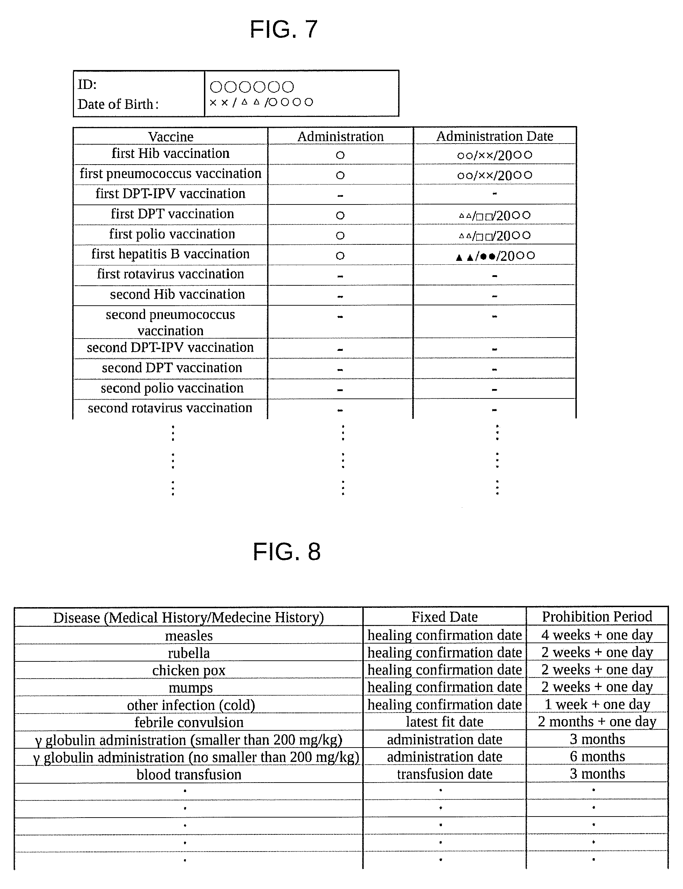

Next, the second storage area (second storage means) is used to store information about vaccines having been administered by each preventive vaccination subject. Fig. 7 is a view showing the stored contents stored in the second storage area. Specifically, the second storage area stores information about the ID number and birth date of each of a plurality of preventive vaccination subjects, information indicating that vaccination has been carried out ("○" in Fig. 7) and information indicating that vaccination has not been carried out yet ("-" in Fig. 7).

Furthermore, with respect to a vaccine having been administered, the second storage area is used to store information about the administration date of the vaccine in association with the vaccine.

Fig. 8 is a view showing the stored contents stored in the third storage area. The third storage area (third storage means) is used to store prohibition periods in which vaccination is prohibited after the fixed dates of a plurality of diseases respectively. In this case, the "disease" is not limited to an infection, but may include a personal medical history such as febrile convulsion, and a medicine history such as blood transfusion and administration of gamma globulin. Also, the fixed date is the date in which healing of an infection was confirmed, the date in which a fit of febrile convulsion finally occurred, the administration date in the case of gamma globulin administration, or the date in which blood transfusion was performed. The fixed date indicates the start point of a prohibition period and differs among the types of diseases. The third storage area is used to store information about fixed dates and prohibition periods in which vaccination is prohibited from the fixed dates respectively in association with "a plurality of diseases".

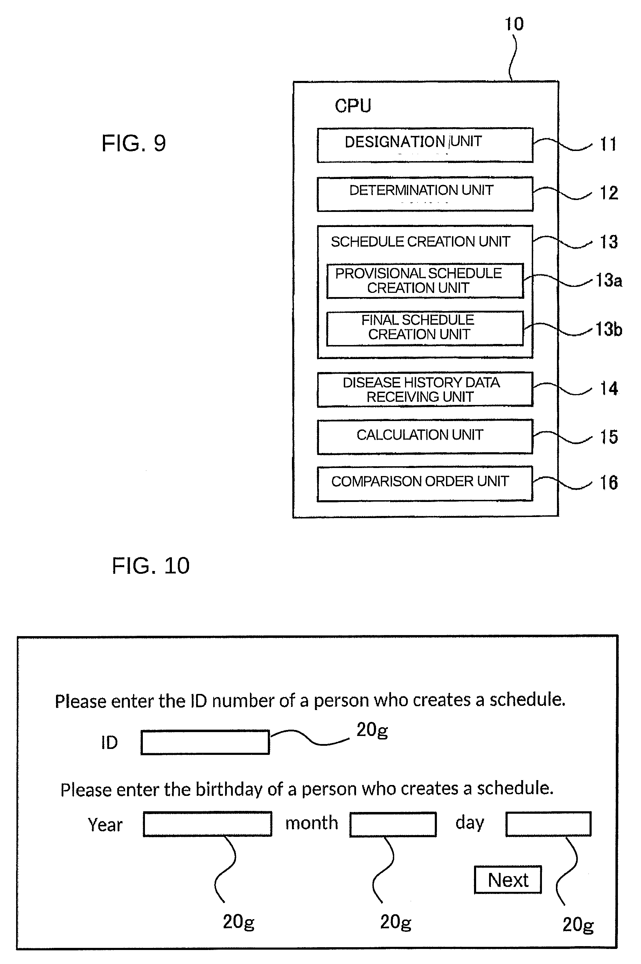

Fig. 9 is a view for showing the functional configuration of the vaccine scheduling device 1 in accordance with the first embodiment. As shown in Fig. 9, the vaccine scheduling device 1 of the first embodiment is provided with, as functions performed by the CPU 10, a designation unit (designation means) 11, a determination unit (determination means) 12, a schedule creation unit (schedule creation means) 13 and a clinical history data receiving unit (clinical history data receiving means) 14.

The designation unit 11 serves to designate either one of a plurality of preventive vaccination subjects as a designated person. Fig. 10 is a third view showing an example of the display screen of the vaccine scheduling device 1 in accordance with the first embodiment. For example, as illustrated in Fig. 10, the display 20 of the vaccine scheduling device 1 displays a screen including input boxes 20g through which an ID number and a date of birth can be input. A user is designated from a plurality of preventive vaccination subjects as a designated person by inputting an ID number and a date of birth through the input boxes 20g and pressing "Next" with this screen being displayed.

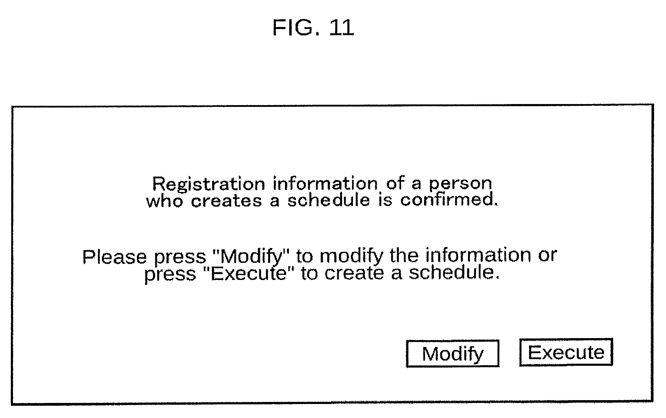

Fig. 11 is a fourth view for showing an example of the display screen of the vaccine scheduling device 1 in accordance with the first embodiment. If input contents are correct, a confirmation screen shown in Fig. 11 is displayed on the display 20. This confirmation screen contains "modify" and "execute" which can be selected, and when "modify" is selected, the screen shown in Fig. 4 is displayed. On the other hand, when "execute" is selected, a schedule creation process is executed.

In creation of a schedule, the determination unit (determination means) 12, the schedule creation unit (schedule creation means) 13 and the clinical history data receiving unit (clinical history data receiving means) 14 serve to function as follows.

First, with respect to the designated person, the determination unit 12 determines simultaneous vaccination or no simultaneous vaccination based on the basic information shown in Fig. 5. In the case where simultaneous vaccination is applicable, the determination unit 12 selects vaccines one by one in descending order of priority levels based on the priority levels of simultaneous vaccination as shown in Fig. 6, and successively determines if vaccination of the selected vaccine is unnecessary based on the basic information shown in Fig. 5 and if the selected vaccine has already been administered with reference to Fig. 7. On the other hand, in the case where simultaneous vaccination is not applicable, the determination unit 12 selects vaccines one by one in descending order of priority levels based on the priority levels of no simultaneous vaccination as shown in Fig. 6, and successively determines if vaccination of the selected vaccine is unnecessary based on the basic information shown in Fig. 5 and if the selected vaccine has already been administered with reference to Fig. 7.

In the case where vaccination of the selected vaccine is not unnecessary and where the selected vaccine has not been administered yet, the determination unit 12 determines the selected vaccine as a vaccine to be administered. On the other hand, if either case is true, the determination unit 12 determines the selected vaccine as a vaccine not to be administered. Thereafter, the above routine is repeated.

The schedule creation unit 13 creates a schedule with vaccines which the determination unit 12 determines as vaccines to be administered to the designated person. This schedule creation unit 13 includes a provisional schedule creation unit (provisional schedule creation means) 13a and a final schedule creation unit (final schedule creation means) 13b as illustrated in Fig. 9.

The provisional schedule creation unit 13a selects vaccines one by one in descending order of priority levels from among vaccines determined by the determination unit 12 as vaccines to be administered to the designated person, and successively determines scheduled administration dates of selected vaccines.

When a schedule is not created at all, if the start date of a schedule to be created is before the start date of the vaccination recommended period of the selected vaccine, this provisional schedule creation unit 13a determines the start date of the vaccination recommended period of the selected vaccine as the scheduled date of the selected vaccine. On the other hand, if the created schedule start date is after the start date of the vaccination recommended period of a selected vaccine and before the end date of the vaccination recommended period or the end date of the vaccination viable period, the provisional schedule creation unit 13a determines the created schedule start date as the scheduled administration date of the selected vaccine.

Incidentally, the created schedule start date is the start date of a schedule to be created, and may be the date when "execute" is pressed in the screen shown in Fig. 11, or the date input to an input box which may be provided in the screen shown in Fig. 11 for inputting the created schedule start date. Furthermore, while the created schedule start date is set as a candidate date of administering the vaccine (candidate administration date), if there is no problem with the scheduled administration date which is set to the created schedule start date as described above, the scheduled administration date of the vaccine is set to the created schedule start date. On the other hand, if there is a problem, the created schedule start date is reset to another date, and if there is no problem with the scheduled administration date which is reset, the scheduled administration date of the vaccine is set to the created schedule start date which is reset.

Also, when at least one vaccine is scheduled (i.e., with respect to at least one vaccine, the scheduled administration date is determined), the provisional schedule creation unit 13a resets the candidate administration date (i.e., the created schedule start date) to the earliest date after the interval period after the scheduled administration date of vaccines administered just before the selected vaccine, and determining the reset candidate administration date as the scheduled administration date of the selected vaccine.

Incidentally, if the scheduled administration date which is determined is before the start date of the vaccination recommended period of the selected vaccine, needless to say, the scheduled administration date is determined to be the start date of the vaccination recommended period of the selected vaccine. Meanwhile, in accordance with the first embodiment, the vaccine which is administered just before is a vaccine having a scheduled administration date determined by the provisional schedule creation unit 13a. However, the present invention is not limited to this, but it may be a vaccine having been administered as shown in Fig. 7. In this case, from the view point of schedule creation, information about administration dates is included in the data as shown in Fig. 7.

Furthermore, in the case where the designated person can receive simultaneous vaccination, with respect to the second and later selected vaccines, the provisional schedule creation unit 13a determines the scheduled administration date of the vaccine to be administered just before the selected vaccine as the scheduled administration date of the selected vaccine until the upper limit number of simultaneous vaccinations is reached. Incidentally, the operation of the provisional schedule creation unit 13a will be described below in detail.

Still further, the created schedule start date will be reset as illustrated in a flow chart which is described in below, and the details thereof will be described below based on the flow chart.

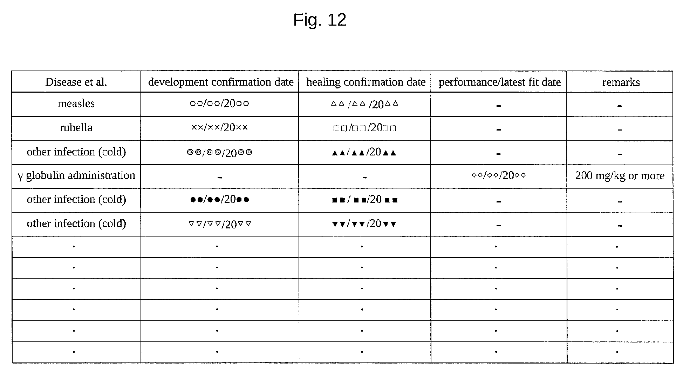

The clinical history data receiving unit 14 receives clinical history data including past diseases of a designated person and the fixed dates thereof. Fig. 12 is a view for showing an example of the clinical history data of a designated person. The HDD 50 and the USB 60 store the clinical history data of the designated person as shown in Fig. 12. That is, the HDD 50 and the USB 60 store the clinical history data of the designated person in a time series which includes, in the case shown in Fig. 12, the measles, the rubella, other infection (cold) (first), gamma globulin administration (more than 200 mg/kg), other infection (cold) (second), and other infection (cold) (third). Then, the HDD 50 and the USB 60 store the fixed dates of these diseases. Namely, there are stored the healing confirmation dates of the measles, the rubella, other infection (cold), and the administration date of gamma globulin administration. Incidentally, although there is no illustration in the example shown in Fig. 12, the date in which a fit of febrile convulsion finally occurred and the date in which blood transfusion was performed may be stored.

Meanwhile, there are a number of patients having cold or the like who do not visit a hospital for the purpose of confirming healing. Because of this, in place of the actual healing confirmation date, a predetermined healing period is added to the date when cold or the like is confirmed at a hospital, and the date after addition is determined as the healing confirmation date.

The clinical history data receiving unit 14 receives clinical history data by reading clinical history data from the HDD 50 and the USB 60 as illustrated in Fig. 12. Incidentally, the clinical history data receiving unit 14 is not only structured to read the clinical history data from the HDD 50 and the USB 60, but also structured to read the clinical history data, for example, from an external computer through the communication Interface unit 30.

The final schedule creation unit 13b reads, from the HDD 50 and the USB 60, the prohibition period corresponding to a past disease contained in the clinical history data of a designated person as received by the clinical history data receiving unit 14, sets the prohibition period from the fixed date contained in the clinical history data, and in the case where the scheduled administration date of a vaccine(s) is between the present time and the last day of the prohibition period in accordance with the provisional schedule created by the provisional schedule creation unit 13a, creates the final schedule by shifting to the future, in accordance with the differential number of days between the earliest scheduled administration date of the vaccine(s) and the release day when the prohibition period ends, the scheduled administration dates of all the vaccines to be administered after the fixed date of the past disease of the clinical history data.

Namely, it is assumed that the provisional schedule creation unit 13a determines, for example, January 10th, 2014 as the first scheduled administration date of Hib vaccine,

January 18th, 2014 as the first scheduled administration date of pneumococcus vaccine, and January 26th, 2014 as the first scheduled administration date of DPT-IPV vaccine. In such a situation, it is assumed that the final schedule creation unit 13b receives clinical history data in which the healing confirmation date of cold is January 12th, 2014 (yesterday). In this case, the final schedule creation unit 13b calculates, as the first scheduled administration date of Hib vaccine, the last day of the prohibition period, i.e., January 20th, 2014 which is one week plus one day after January 12th, 2014. Next, the final schedule creation unit 13b confirms that the first scheduled administration date of pneumococcus vaccine is between January 13th, 2014 which is today and January 20th, 2014 which is the last day of the prohibition period. Then, the final schedule creation unit 13b shifts the first scheduled administration date of pneumococcus vaccine and the first scheduled administration date of DPT-IPV vaccine after January 12th, 2014 which is the healing confirmation date (fixed date) by three days as the differential number of days (January 21 (the day after the last day of the prohibition period). By this configuration, the final schedule creation unit 13b determines January 21nd, 2014 as the first scheduled administration date of pneumococcus vaccine, and January 29th, 2014 as the first scheduled administration date of DPT-IPV vaccine.

As has been discussed above, the vaccine scheduling device 1 can create a preventive vaccination schedule by taking into consideration clinical history data, and prepare a preventive vaccination schedule which is suitable for a preventive vaccination subject.

Fig. 9 is referred to again. As illustrated in Fig. 9, the vaccine scheduling device 1 in accordance with the first embodiment is further provided, as functions performed by the CPU 10, with a calculation unit (calculation means) 15 and a comparison order unit (comparison order means) 16.

The calculation unit 15 collects final schedules created by the final schedule creation unit 13b for a plurality of preventive vaccination subjects, and calculates the types (i.e., vaccine names) and numbers of vaccines to be administered in a predetermined period. In this case, preferably, the plurality of preventive vaccination subjects are all the persons for which the schedules are created in a medical facility. Also, preferably, the predetermined period can readily be modified by operating the input means of the vaccine scheduling device 1.

The comparison order unit 16 compares the types and numbers of vaccines to be administered in the predetermined period as calculated by the calculation unit 15 with the types and numbers of vaccines in stock, and orders the shortage amounts. The shortage amount is not limited to the number of vaccines to be administered minus the number of vaccines in stock, but may be the number of vaccines to be administered plus α (an arbitrary positive integer) minus the number of vaccines in stock. This is because, even if someone suddenly appears and desires preventive vaccination on that very day, the number α makes it possible to perform the vaccination. Also, the order may be done by automatically transmitting through the communication Interface unit 30, or by preparing order data and transmitting or printing out the order data by an operator.

More specifically described, the comparison order unit 16 determines one of vaccines to be administered in the predetermined period, and performs comparison with this one vaccine. Namely, with respect to a determined vaccine, the comparison order unit 16 calculates the shortage amount by performing an arithmetic operation to obtain the number of vaccines to be administered in the predetermined period minus the number of vaccines in stock. The comparison order unit 16 then repeats similar calculation of the shortage amounts for the other vaccines than the determined vaccine by performing an arithmetic operation to obtain the number of vaccines to be administered in the predetermined period minus the number of vaccines in stock. Next, the comparison order unit 16 generates the information for associating the types of vaccines with the shortage amounts respectively, and makes orders by transmitting the information to a predetermined institution.

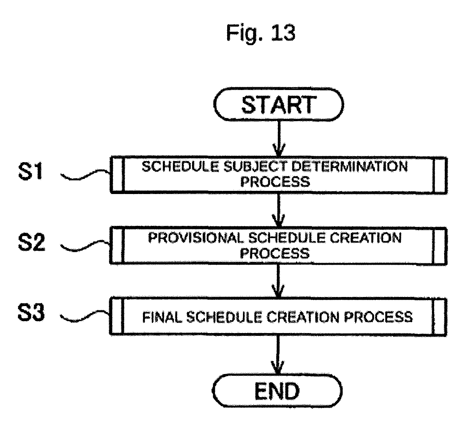

Next, a schedule creation method by the vaccine scheduling device 1 in accordance with the first embodiment will be explained in detail. Fig. 13 is a flow chart for showing the schedule creation method by the vaccine scheduling device 1 in accordance with the first embodiment. It is assumed first that "execute" shown in Fig. 11 is pressed after inputting an ID number and the like shown in Fig. 10. Namely, it is assumed that a person is designated.

The determination unit 12 thereby performs a schedule subject determination process (S1). Vaccines to be administered to the designated person are determined of a number of vaccines by this schedule subject determination process.

Next, with respect to the vaccines determined in step S1 to be administered, the provisional schedule creation unit 13a performs a provisional schedule creation process to create a provisional schedule (S2). The final schedule creation unit 13b then performs a final schedule creation process with the provisional schedule created in step S2 to create a final schedule (S3).

The final schedule which is created is displayed on the display 20, transmitted as data to the designated person, the guardian thereof and so on through the communication Interface unit 30.

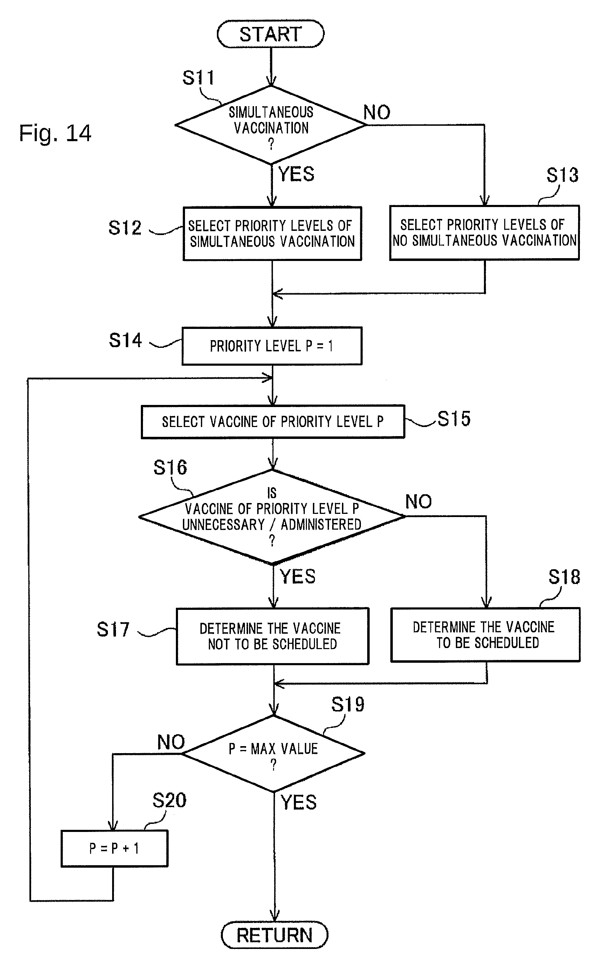

Fig. 14 is a flow chart for showing the schedule subject determination process (S1) shown in Fig. 13 in detail. As illustrated in Fig. 14, first, the determination unit 12 determines whether or not simultaneous vaccination is applicable to the designated person based on the basic information shown in Fig. 5 (S11). If it is determined that simultaneous vaccination is applicable (S11: YES), the determination unit 12 selects the priority levels of simultaneous vaccination shown in Fig. 6 (S12), and the process proceeds to step S14. Conversely, if it is not determined that simultaneous vaccination is applicable (S11: NO), the determination unit 12 selects the priority levels of no simultaneous vaccination shown in Fig. 6 (S13), and the process proceeds to step S14.

In step S14, the determination unit 12 initializes a priority level P to "1" (S14). Next, based on the priority levels selected in step S12 or S13, the determination unit 12 selects a vaccine of the priority level P (S 15). The determination unit 12 then determines whether or not the vaccine of the priority level P is described in the basic information shown in Fig. 5 as unnecessary, and whether or not the vaccine of the priority level P has been administered with reference to the information about vaccines shown in Fig. 7 (S16).

If it is determined that the vaccine is unnecessary or has been administered (S16: YES), the determination unit 12 determines the vaccine of the priority level P as not to be entered in the schedule (S17), and the process proceeds to step S19. Conversely, if it is determined that the vaccine is neither unnecessary nor has been administered (S16: NO), the priority level P determines the vaccine of the priority level P as an entry of the schedule (S18), and the process proceeds to step S19.

In step S19, the determination unit 12 determines whether or not the priority P reaches a MAX value (S19). If it is determined that the priority P does not reach the MAX value (S19: NO), the determination unit 12 increments the value of the priority level P (S20), and the process proceeds to step S 15. Contrary to this, if it is determined that the priority P reaches the MAX value (S19: YES), the routine shown in Fig. 14 is completed and the process proceeds to the provisional schedule creation process (S2) shown in Fig. 13.

Incidentally, although not shown in the figure, it is needless to say that, with respect to the priority level P = 9 when simultaneous vaccination is applicable and the priority levels P = 8 and 15 when simultaneous vaccination is not applicable, the process skips steps S16 to S19 and proceeds to step S20.

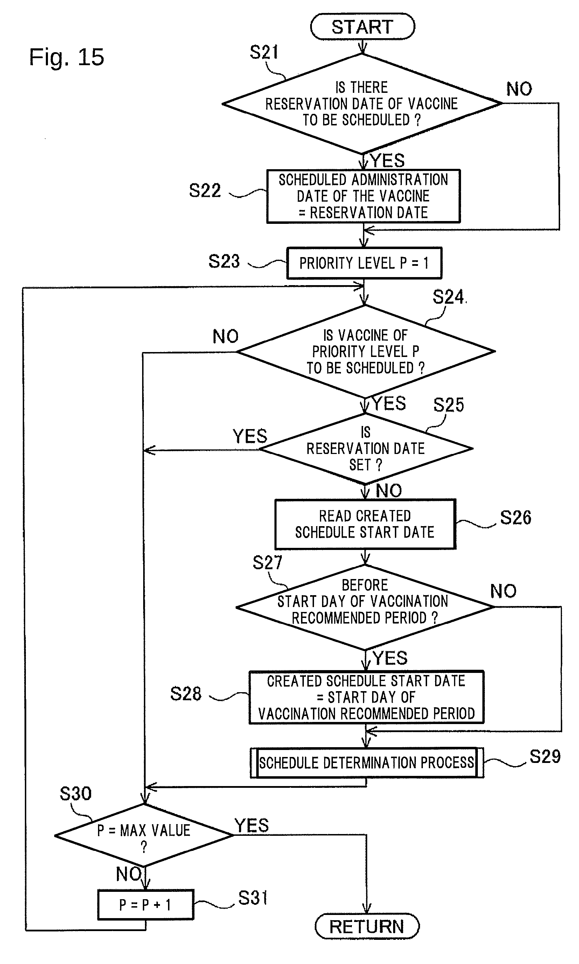

Fig. 15 is a flow chart for showing the provisional schedule creation process (S2) shown in Fig. 13 in detail. As illustrated in Fig. 15, first, the provisional schedule creation unit 13a determines whether or not there is the day for group vaccination in a local government or the scheduled administration date of a particular vaccine designated by a designated person or a guardian (hereinafter referred to as a reservation date) (S21). Although not shown in Fig. 5, this reservation date is to be stored in the basic information shown in Fig. 5 through the communication Interface unit 30 or the input means such that its date information is stored in the columns 20e indicating unnecessary or the like. Also, in the case where this reservation date is the day for group vaccination, even if the basic information shown in Fig. 5 indicates that simultaneous vaccination is applicable, the simultaneous vaccination is practically impossible (because group vaccination is carried out in a healthcare center or the like) and therefore the reservation date is treated in the same manner as in the case where simultaneous vaccination is not applicable in a flow chart to be described below.

Fig. 15 is referred to again. If it is determined that there is a reservation date (S21: YES), the scheduled administration date of the vaccine is set to the reservation date (S22), and the process proceeds to step S23. If it is determined that there is no reservation date (S21: NO), the process proceeds to step S23 after skipping step S22.

In step S23, the provisional schedule creation unit 13a initializes the priority level P to "1" (S23). Next, the provisional schedule creation unit 13a determines whether or not the vaccine of the priority level P is to be scheduled (S24). In this case, the provisional schedule creation unit 13a determines that the vaccine is to be scheduled based on the information determined in steps S 17 and S18.

If it is determined that the vaccine of the priority level P is to be scheduled (S24: YES), the provisional schedule creation unit 13a determines whether or not a reservation date is set for the vaccine of the priority level P (S25). If it is determined that no reservation date is set (S25: NO), step S22 has been skipped so that the process proceeds to steps S26 to S29 in order to schedule the vaccine of the priority level P.

In step S26, the provisional schedule creation unit 13a reads information about the created schedule start date as described above (S26). The candidate administration date of the vaccine of the priority level P is thereby set up. The provisional schedule creation unit 13a then determines whether or not the created schedule start date is before the start day of the vaccination recommended period of the vaccine of the priority level P (S27). Meanwhile, as illustrated in Fig. 5, the HDD 50 and USB 60 store the date of birth. Because of this, based on information about the date of birth and information about the vaccination recommended period shown in Fig. 6, the provisional schedule creation unit 13a calculates the starting date of the vaccination recommended period and performs determination in step S27. On the other hand, with respect to influenza and synagis, the starting date of the vaccination recommended period need not be calculated, but information about the vaccination recommended period shown in Fig. 6 is utilized as it is. Furthermore, in the case where the first rotavirus vaccination has been finished or the scheduled administration date of the first rotavirus vaccination has been determined (inclusive of determination by this device, and hospital reservation), the vaccination recommended period of the second rotavirus vaccination is calculated with reference to the administration date (or scheduled administration date) of the first rotavirus vaccination. Namely, the vaccination recommended period is calculated with reference to the previous administration date of the same type of vaccine. Incidentally, also in the following process, it is needless to say that the starting date and the ending date are calculated with reference to information about the date of birth, information about the previous administration date (scheduled administration date) and information about various periods.

If it is determined that the created schedule start date is not before the start day of the vaccination recommended period of the vaccine of the priority level P (S27: NO), the process proceeds to step S29. Conversely, if it is determined that the created schedule start date is before the start day of the vaccination recommended period of the vaccine of the priority level P (S27: YES), the provisional schedule creation unit 13a reset the created schedule start date to the start day of the vaccination recommended period of the vaccine of the priority level P, and the process proceeds to step S29.

Then, the provisional schedule creation unit 13a performs a schedule determination process (S29). By this process, the scheduled administration date of the vaccine of the priority level P is determined. The process then proceeds to step S30.

Incidentally, in the case where the vaccine of the priority level P is not to be scheduled (S24: NO) or where there is the reservation date for the vaccine of the priority level P (S25: YES), the process proceeds to step S30.

In step S30, the provisional schedule creation unit 13a determines whether or not the priority P reaches the MAX value (S30). If it is determined that the priority P does not reach the MAX value (S30: NO), the provisional schedule creation unit 13a increments the value of the priority level P (S31), and the process proceeds to step S24. Contrary to this, if it is determined that the priority P reaches the MAX value (S30: YES), the routine shown in Fig. 15 is completed and the process proceeds to the final schedule creation process (S3) shown in Fig. 13.

Incidentally, although not shown in the figure, it is needless to say that, with respect to the priority level P = 9 when simultaneous vaccination is applicable and the priority levels P = 8 and 15 when simultaneous vaccination is not applicable, the process skips steps S24 to S30 and proceeds to step S31.

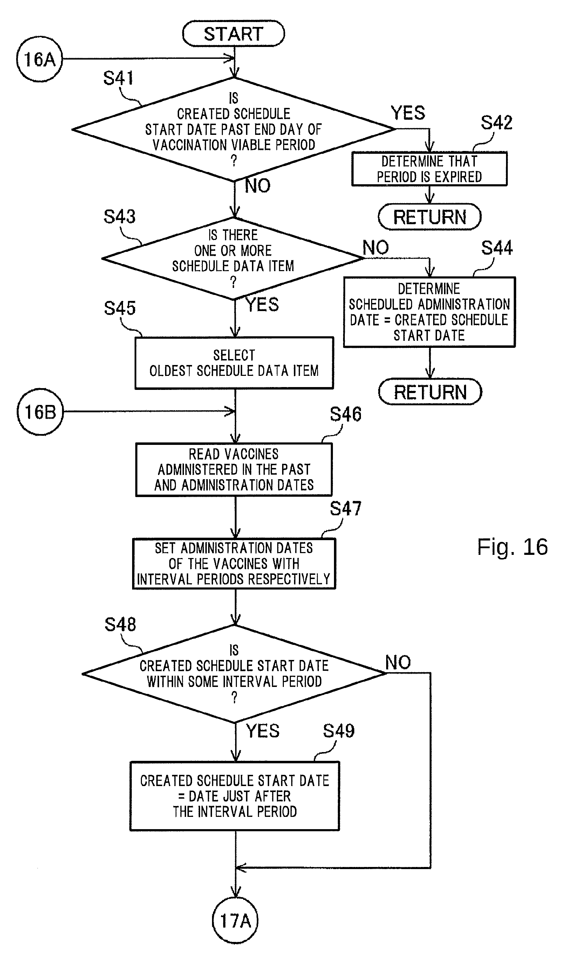

Fig. 16 through Fig. 19 are a flow chart showing the schedule determination process (S29) shown in Fig. 15 in detail. As illustrated in Fig. 16, first, the provisional schedule creation unit 13a determines whether or not the created schedule start date is past the end day of the vaccination viable period of the vaccine of the priority level P (S41). If it is determined that the created schedule start date is past the end day (S41: YES), the provisional schedule creation unit 13a determines that the period is expired with respect to the vaccine of the priority level P (S42), and the routine shown in Fig. 16 through Fig. 19 is finished. The process then proceeds to step S30 shown in Fig. 15.

Contrary to this, if it is determined that the created schedule start date is not past the end day (S41: NO), the provisional schedule creation unit 13a determines whether or not there is one or more schedule data item (S43). The schedule data item is a data item consisting of information about the name of a vaccine to be administered as preventive vaccination and information about the scheduled administration date of the vaccine in association with each other.

If it is determined that there is no schedule data item (S43: NO), the provisional schedule creation unit 13a determines the created schedule start date as the scheduled administration date of the vaccine of the priority level P (S44), and the routine shown in Fig. 16 through Fig. 19 is finished after creating the schedule data item. The process then proceeds to step S30 shown in Fig. 15.

Conversely, if it is determined that there is one or more schedule data item (S43: YES), the provisional schedule creation unit 13a selects the oldest schedule data item (S45). In this case, the oldest schedule data item is the schedule data item having a scheduled administration date of a vaccine which is the earliest of the schedule data items which have been created by the vaccine scheduling device 1.

Next, the provisional schedule creation unit 13a reads the information shown in Fig. 7, i.e., the information about vaccines administered in the past and the information about the administration dates of the vaccines (S46). Next, the provisional schedule creation unit 13a sets the administration dates of vaccines having been administered with the interval period of these vaccines respectively (S47).

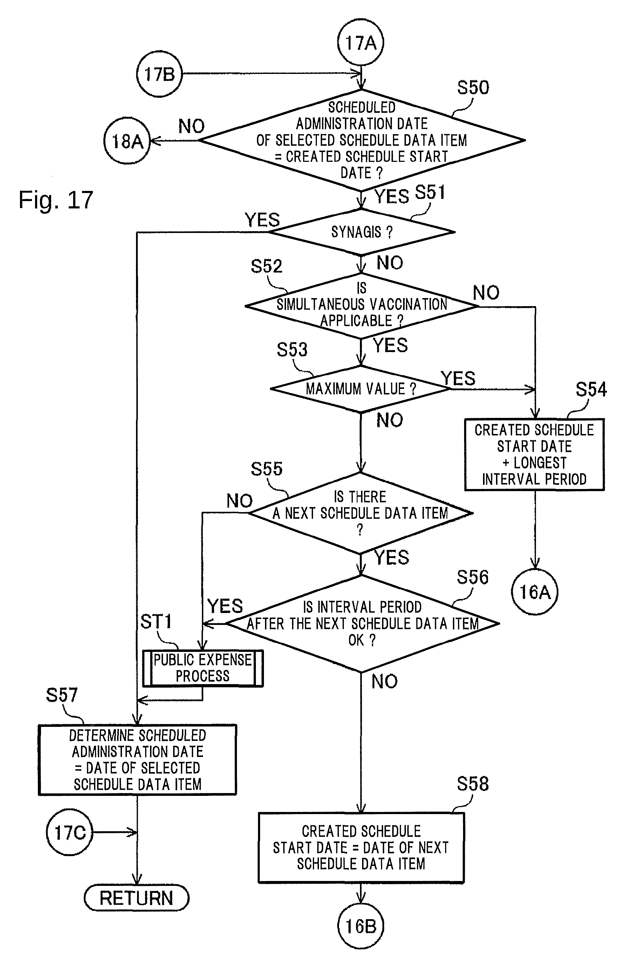

The provisional schedule creation unit 13a then determines whether or not there is any one of these interval periods within which the created schedule start date falls (S48). If it is determined that there is no such an interval period (S48: NO), the process proceeds to step S50 shown in Fig. 17. Conversely, if it is determined that there is such an interval period (S48: YES), the provisional schedule creation unit 13a sets the created schedule start date to the date just after the interval period of the vaccine within which the created schedule start date is determined to fall (S49). The process then proceeds to step S50 shown in Fig. 17.

In step S50 of Fig. 17, the provisional schedule creation unit 13a determines whether or not the scheduled administration date of the vaccine of the selected schedule data item is the same date as the created schedule start date.

If it is determined that these dates are the same (S50: YES), the provisional schedule creation unit 13a determines whether or not the vaccine of the priority level P is the synagis vaccine (S51). If it is determined that the vaccine is the synagis vaccine (S51: YES), the provisional schedule creation unit 13a determines the scheduled administration date of the synagis vaccine as the scheduled administration date of the selected schedule data item (S57). The provisional schedule creation unit 13a then create a schedule data item of the synagis vaccine, and terminates the routine shown in Fig. 16 through Fig. 19. Next, the process proceeds to step S30 shown in Fig. 15.

On the other hand, if it is determined that the vaccine is not the synagis vaccine (S51: NO), the provisional schedule creation unit 13a determines whether or not simultaneous vaccination is applicable to the designated person based on the basic information shown in Fig. 5 (S52). If simultaneous vaccination is applicable (S52: YES), the provisional schedule creation unit 13a determines whether or not the number of vaccinations of the selected schedule data item reaches the maximum value of simultaneous vaccinations based on the basic information shown in Fig. 5 (S53).

Incidentally, in the case where simultaneous vaccination is applicable, schedule data items are sometimes created by associating information about a plurality of names of vaccines to be administered with information about the scheduled administration date of these vaccines. In the process of step S53, the provisional schedule creation unit 13a determines, when schedule data items are created in which, for example, the first Hib vaccine and the first pneumococcus are to be simultaneously administered in ○○/○○/20○○, the number of vaccinations of the selected schedule as "2", and determines whether or not this number reaches the maximum value of simultaneous vaccinations.

If it is determined that the number does not reach the maximum value of simultaneous vaccinations (S53: NO), the process proceeds to step S55. On the other hand, in the case where it is determined that simultaneous vaccination is not applicable (S52: NO), it is not possible to add a new vaccine to the scheduled administration date of the selected schedule data item as shown in step S57 because simultaneous vaccination cannot be performed. Accordingly, the process proceeds to step S54, and the provisional schedule creation unit 13a adds the interval period of the vaccine of the selected schedule data item to the created schedule start date, and reset the created schedule start date to this date (i.e., the last day after the interval period is added) (S54). The process then proceeds to step S41 of Fig. 16.

Likewise, if it is determined that the number reaches the maximum value of simultaneous vaccinations (S53: YES), a vaccine to be simultaneously administered can no longer be added to the selected schedule data item, and therefore the provisional schedule creation unit 13a adds the longest interval period to the scheduled administration date of the selected schedule data item, and reset the created schedule start date to this date (i.e., the last day after adding the longest interval period of the vaccines to be administered to the scheduled administration date) (S54). The process then proceeds to step S41 of Fig. 16.

In step S55, the provisional schedule creation unit 13a determines whether or not there is a next schedule data item (i.e., the schedule data item having a scheduled administration date which is set in the future subsequent to the scheduled administration date of the selected schedule data item) (S55).

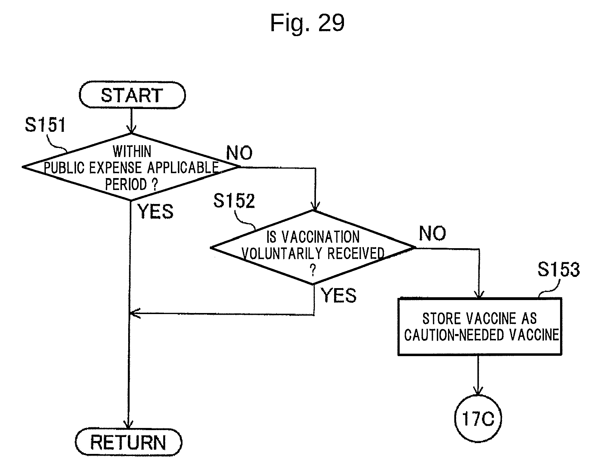

If it is determined that there is no next schedule data item (S55: NO), the provisional schedule creation unit 13a performs a public expense process (ST1). Fig. 29 is a flow chart for showing the public expense process to be performed in step ST1 of Fig. 17 in detail. As illustrated in Fig. 29, first, the provisional schedule creation unit 13a determines whether or not the created schedule start date of the vaccine of the priority level P falls within the applicable period of the public expense as shown in Fig. 6 (S 151). If it is determined that the date falls within the applicable period of the public expense (S 151: YES), the process proceeds to step S57 shown in Fig. 17.

Conversely, if it is determined that the date does not fall within the applicable period of the public expense (S 151: YES), the provisional schedule creation unit 13a determines whether or not vaccination is voluntarily received (S151). In this case, whether or not vaccination is voluntarily received is determined based on the basic information shown in Fig. 5. If it is determined that vaccination is voluntarily received (S 152: YES), the process proceeds to step S57 shown in Fig. 17.

Conversely, if it is determined that vaccination is not voluntarily received (S 152: NO), the provisional schedule creation unit 13a stores the vaccine of the priority level P as a caution-needed vaccine (S153), and the process proceeds to step S30 shown in Fig. 15. In this case, the caution-needed vaccine is a vaccine which is not administered. The vaccine scheduling device 1 is structured to make it possible to refer to such a caution-needed vaccine, for example, after creation of a schedule and provide a reminder.

Fig. 17 is referred to again. In step S57 of Fig. 17, the provisional schedule creation unit 13a adds the vaccine of the priority level P to the scheduled administration date of the selected schedule data item (S57), and after creating a schedule data item, the routine shown in Fig. 16 through Fig. 19 is completed. The process then proceeds to step S30 shown in Fig. 15.

If there is a next schedule data item (S55: YES), the provisional schedule creation unit 13a determines whether or not a sufficient interval is secured with the next schedule data item after adding the vaccine of the priority level P to the scheduled administration date of the selected schedule data item (S56). Namely, after adding the vaccine of the priority level P to the scheduled administration date of the selected schedule data item, the longest interval period of the vaccines to be administered in the scheduled administration date is added to the created schedule start date, and it is determined whether or not this (i.e., the last day after the longest interval period is added) is in a future time later than the scheduled administration date of the next schedule data item.

If a sufficient interval is secured (S56: YES), the provisional schedule creation unit 13a adds the vaccine of the priority level P to the scheduled administration date of the selected schedule data item (S57) after the public expense process (ST1), and the routine shown in Fig. 16 through Fig. 19 is completed after creating a schedule data item. The process then proceeds to step S30 shown in Fig. 15.

Conversely, if a sufficient interval is not secured (S56: NO), the vaccine of the priority level P cannot be added to the scheduled administration date of the selected schedule data item, the provisional schedule creation unit 13a thereby resets the created schedule start date to the scheduled administration date of the next schedule data item (S58), and the process proceeds to step S46 of Fig. 16.

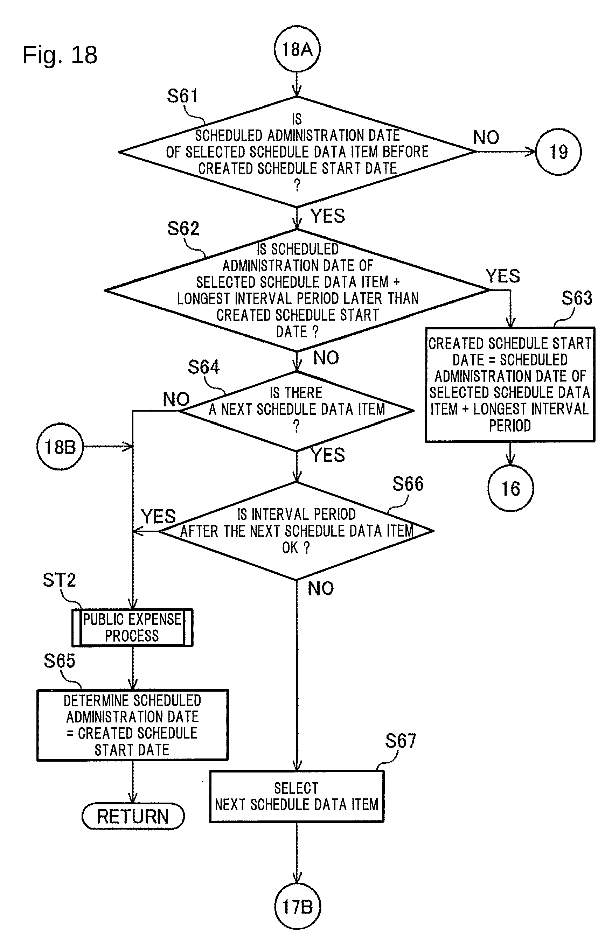

Incidentally, if it is determined that the scheduled administration date of the selected schedule data item is not the same date as the created schedule start date (S50: NO), the process proceeds to Fig. 18.

In Fig. 18, first, the provisional schedule creation unit 13a determines whether or not the scheduled administration date of the selected schedule data item is before the created schedule start date (S61). If it is determined that the scheduled administration date is before (S61: YES), the provisional schedule creation unit 13a adds the longest interval period of the vaccine of the selected schedule data item to the scheduled administration date of the selected schedule data item, and determines whether or not this (i.e., the last day after the longest interval period is added) is in a future time later than the created schedule start date (S62).

If it is determined that the last day is in a future time (S62: YES), the provisional schedule creation unit 13a resets the created schedule start date to the date after the addition in step S62, and the process proceeds to step S41 shown in Fig. 16.

Conversely, if it is determined that the last day is not in a future time (S62: NO), the provisional schedule creation unit 13a determines whether or not there is a next schedule data item (S64). If it is determined that there is no next schedule data item (S64: NO), the provisional schedule creation unit 13a performs the public expense process (ST2). This public expense process (ST2) is the same as the above public expense process (ST1), and therefore explanation is not repeated. The provisional schedule creation unit 13a then determines the created schedule start date as the scheduled administration date of the vaccine of the priority level P (S65), and the routine shown in Fig. 16 through Fig. 19 is finished after creating the schedule data item. The process then proceeds to step S30 shown in Fig. 15.

Namely, in the process of step S65, the created schedule start date is in a future time later than the scheduled administration date of the selected schedule data item, and also later than the scheduled administration date of the selected schedule data item to which the longest interval period is added. Furthermore, since there is no next schedule data item subsequent to the selected schedule data item, there is no problem even if the scheduled administration date of the vaccine of the priority level P is set to the created schedule start date. Accordingly, the provisional schedule creation unit 13a determines the created schedule start date as the scheduled administration date of the vaccine of the priority level P.

On the other hand, if it is determined that there is a next schedule data item (S64: YES), it is determined whether or not a sufficient interval is secured before the scheduled administration date of the next schedule data item (S66). Namely, when the created schedule start date is determined to be the scheduled administration date of the vaccine of the priority level P as shown in step S65, the provisional schedule creation unit 13a adds the interval period of the vaccine to this scheduled administration date, and determines whether or not this date (i.e., the last day after the interval period is added) is in a future time later than the scheduled administration date of the next schedule data item.

If it is determined that there is a sufficient interval (S66: YES), the provisional schedule creation unit 13a determines the created schedule start date as the scheduled administration date of the vaccine of the priority level P (S65), and the routine shown in Fig. 16 through Fig. 19 is completed after creating a schedule data item. The process then proceeds to step S30 shown in Fig. 15.

Conversely, if it is determined that there is no sufficient interval period (S66: NO), the provisional schedule creation unit 13a selects the next schedule data item (S67), and the process proceeds to step S50 shown in Fig. 17. Namely, in the case where there is no sufficient interval period, if the created schedule start date were determined as the scheduled administration date of the vaccine of the priority level P as illustrated in step S65, the preventive vaccination cannot be received in the scheduled administration date of the next schedule data item, and therefore the provisional schedule creation unit 13a selects the next schedule data item.

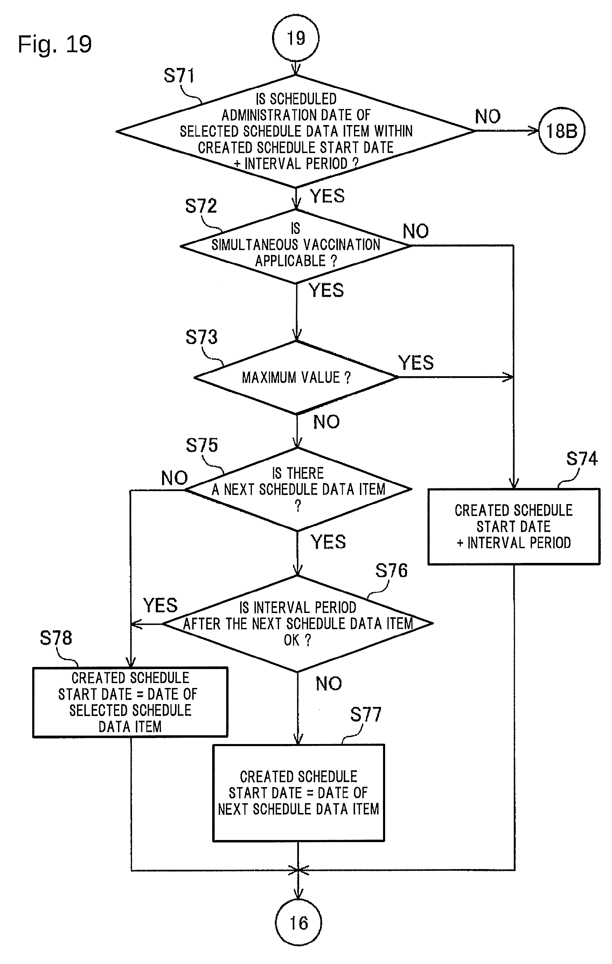

Incidentally, if the scheduled administration date of the selected schedule data item is not before the created schedule start date (S61: NO), i.e., if the scheduled administration date of the selected schedule data item is in a future time, the process proceeds to Fig. 19.

In Fig. 19, first, the provisional schedule creation unit 13a determines whether or not the scheduled administration date of the selected schedule data item is within the interval period of the vaccine of the priority level P from the created schedule start date (S71). If it is determined that the scheduled administration date of the selected schedule data item is not within the interval period of the vaccine of the priority level P from the created schedule start date (S71: NO), the process proceeds to step S65 of Fig. 18.

Conversely, if it is determined that the scheduled administration date of the selected schedule data item is within the interval period of the vaccine of the priority level P from the created schedule start date (S71: YES), the provisional schedule creation unit 13a performs the process of steps S72 through S77. This process of steps S72 through S77 is similar to the process of steps S52 through S57 shown in Fig. 16.

Then, if it is determined that there is no next schedule data item (S75: NO), and if a sufficient interval is secured (S76: YES), the provisional schedule creation unit 13a sets the created schedule start date to the scheduled administration date of the selected schedule data item (S78), and the process proceeds to step S41 shown in Fig. 16.

Meanwhile, in step S78 unlike step S58, the scheduled administration date of the selected schedule data item is not determined as the scheduled administration date, but first the created schedule start date is reset to the scheduled administration date of the selected schedule data item followed by performing the process of steps S52 through S58 again.

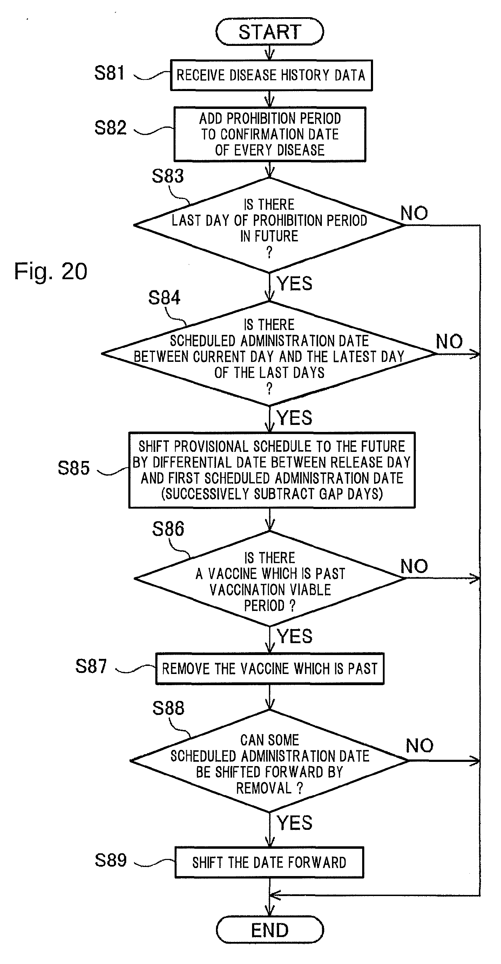

Fig. 20 is a flow chart for showing the final schedule creation process (S3) shown in Fig. 13 in detail. As illustrated in Fig. 20, first, the clinical history data receiving unit 14 receives clinical history data of a designated person (S81).

Next, the final schedule creation unit 13b adds the prohibition period of every disease contained in the clinical history data received in step S81 to the fixed date of the every disease (S82). The final schedule creation unit 13b then determines whether or not the prohibition periods include a period, the last day of which is in the future (S83). Meanwhile, the future in step S83 includes the current day.

If it is determined that there is no prohibition period in the future (S83: NO), the process shown in Fig. 20 is finished. Namely, the provisional schedule created by the provisional schedule creation unit 13a is a final schedule as it is.

Conversely, if it is determined that there is a prohibition period in the future (S83: YES), the final schedule creation unit 13b determines whether or not there is a scheduled administration date between the current day and the latest day of the last days of the prohibition periods which are determined as in the future in step S83 (S84). In this process, the final schedule creation unit 13b determines whether or not a scheduled administration date therebetween with reference to the provisional schedule created by the provisional schedule creation unit 13a. Incidentally, the term "therebetween" includes the case where the current day and the latest day are the same day.

If it is determined that there is no scheduled administration date therebetween (S84: NO), the process shown in Fig. 20 is finished. Namely, the provisional schedule created by the provisional schedule creation unit 13a is a final schedule as it is.

If it is determined that there is a scheduled administration date therebetween (S84: YES), the final schedule creation unit 13b creates the final schedule creation unit by shifting the provisional schedule to the future in accordance with the differential date (for example, by the differential date) between the above release day and the first (the closest future from the current time) scheduled administration date (S85).

The final schedule creation unit 13b then determines whether or not, of the vaccines of the schedule data items in the final schedule creation unit created in step S85, there is a vaccine which is past the last day of the vaccination viable period (S86).

If it is determined that there is no vaccine which is past the last day, the process shown in Fig. 20 is finished. Namely, the final schedule creation unit created in step S85 is employed.

Conversely, if it is determined that there is a vaccine which is past the last day, it is determined that the use period of the vaccine is expired, and this vaccine is removed from the provisional schedule (S87).

The final schedule creation unit 13b then determines whether or not some scheduled administration date can be shifted forward in the light of the removal (S88). In this case, first, the removed vaccine is taken into consideration for determining whether or not some scheduled administration date can be shifted forward. Namely, in the case where the removed vaccine is not simultaneously administered, this vaccine is administered alone so that it is possible to shift the scheduled administration dates of vaccines scheduled after the removed vaccine to the past side by the interval period of the removed vaccine. Furthermore, in the case where the removed vaccine is simultaneously administered and has a longer interval period than other vaccines which were to be simultaneously administered with the removed vaccine, it is possible to shift the scheduled administration dates of vaccines scheduled after the removed vaccine to the past side by the differential date between the interval period of the removed vaccine and the interval periods of the other vaccines.

However, needless to say, the vaccines to be shifted have not to be before the start days of the vaccination recommended periods thereof respectively when shifted to the past side.

If it is determined from the above scenario that some scheduled administration date can be shifted forward (S88: YES), the final schedule creation unit 13b shifts forward the scheduled administration dates of vaccines scheduled after the removed vaccine by the above days (S89). The final schedule creation unit 13b then determines this schedule as a final schedule, the process shown in Fig. 20 is finished.

Conversely, if it is determined that no scheduled administration date can be shifted forward (S88: NO), the final schedule creation unit 13b determines the schedule from which the expired vaccine is removed in step S87 as a final schedule, and the process shown in Fig. 20 is finished.

Incidentally, while a scheduled administration date is shifted to the future in accordance with the above differential date in the case of the above scenario of creating a final schedule, however, if the scheduled administration date is the reservation date of group vaccination, this date cannot be shifted. Because of this, the final schedule creation unit 13b may delete the scheduled administration date of group vaccination, abandon the group vaccination of the vaccines to be group vaccinated (i.e., delete the reservation date), and return the process to step S2 shown in Fig. 13.

Also, when shifting the schedule to the future in accordance with the above differential date in step S85, it is desirable to take the number of gap days into consideration. In this case, the number of gap days are days between the day just after the interval period elapses from the scheduled administration date of a certain vaccine, and the scheduled administration date of the next vaccine to be administered just after the certain vaccine.

For example, it is assumed that the third DPT-IPV vaccine and the third hepatitis B vaccine are to be simultaneously administered in the first day of five months after birth. In addition, it is assumed next that the fourth Hib vaccine, the fourth pneumococcus vaccine and the fourth DPT-IPV vaccine are to be simultaneously administered in the first day of twelve months after birth. In this case, the number of gap days corresponds to the differential date between the first day of twelve months after birth and the day one week after the first day of five months after birth. Because of this, in the case where the differential date in step S85 is about two months, the scheduled administration date is shifted to the future in order that the third DPT-IPV vaccine and the third hepatitis B vaccine are simultaneously administered in the first day of seven months after birth. On the other hand, the fourth Hib vaccine, the fourth pneumococcus vaccine and the fourth DPT-IPV vaccine are to be simultaneously administered in the first day of twelve months without shifting the scheduled administration dates.

Namely, with respect to a vaccine to be administered in the future from the current date, in the case where there are a number of gap days between the day just after the interval period elapses from the scheduled administration date of this vaccine and the scheduled administration date of the next vaccine to be administered just after this vaccine, the scheduled administration date is shifted by the differential date from which the number of gap days are subtracted.

For further example, it is assumed that there are vaccines A to D to be administered after the fixed dates of the clinical history data, and that the differential date in step S85 is ten days. Also, it is assumed that the number of the gap days α between the vaccine A and the vaccine B is four, that the number of the gap days β between the vaccine B and the vaccine C is three, and that the number of the gap days between the vaccine C and the vaccine D is four. In this case, while the scheduled administration date of the vaccine A is shifted to the future by ten days, the scheduled administration date of the vaccine B is shifted to the future only by six days which is the differential date (ten days) minus the number of the gap days α (four days). Furthermore, with respect to the vaccine C, the number of the gap days β is three corresponding to the vaccine B through the vaccine C, so that the scheduled administration date is shifted to the future only by three days which is the differential date (ten days) minus the number of the gap days α (four days) minus the number of the gap days β (three days). Then, with respect to the vaccine D, since there are eleven gap days as the sum of the numbers of the gap days from the vaccine A through the vaccine C, the differential date ≦ the numbers of the gap days, and therefore the scheduled administration date is not shifted.

Accordingly, with respect to each of all the vaccines to be administered after the fixed dates of the clinical history data, the final schedule creation unit 13b calculates the number of days by subtracting the number of gap days from the differential date, and shifts the scheduled administration date to the future by the calculated number of days if this calculated number of days is positive. By this configuration, even if a disease has been developed, it is possible to create an appropriate schedule by making effective use of the number of gap days.

Incidentally, while the process is not limited to the above, the final schedule creation unit 13b can perform two stages of processing by first shifting the scheduled administration date to the future by the differential date, and then shifting the scheduled administration date forward by the number of gap days. Namely, the final schedule creation unit 13b shifts the scheduled administration date of the vaccines to be administered after the fixed dates of the clinical history data to the future by the differential date, and thereafter calculates the number of gap days of each of all the vaccines having been shifted to the future, and shifts to the past the scheduled administration dates of all the vaccines having been shifted to the future, by the number of gap days in a range not exceeding the differential date.

As has been discussed above, for example, these two procedures can be employed to create a final schedule by the use of the differential date and the number of gap days. Accordingly, the final schedule creation unit 13b serves as a final schedule creation means which creates a final schedule in accordance with the differential date. Alternatively, the final schedule creation unit 13b may shift the scheduled administration date of the vaccines to be administered after the fixed dates to the future only by the differential date without taking into consideration the number of gap days.

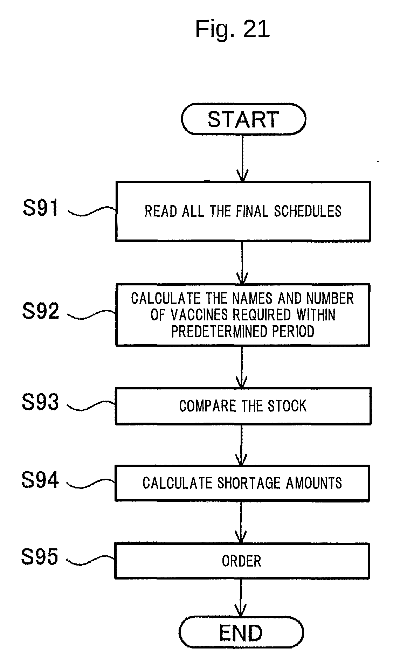

Fig. 21 is a flow chart for showing the process of the vaccine scheduling device 1 in accordance with the first embodiment and showing the process of ordering vaccines.

As illustrated in Fig. 21, first, the calculation unit 15 reads the final schedules of all the preventive vaccination subjects to be vaccinated in their medical facilities based on the information about designated medical facilities shown in Fig. 5 (S91). Next, the calculation unit 15 calculates the names and number of vaccines required within a predetermined period with reference to all final schedules which are read (S92).

Next, the comparison order unit 16 calculate shortage amounts (S94) by comparing the stock (S93) with the names and number of vaccines required within the predetermined period and calculated in step S92. Needless to say, the shortage amount is calculated for each of the names of vaccines. Also, the shortage amount may be the number of vaccines to be administered in the predetermined period minus the number of vaccines in stock, or the number of vaccines to be administered in the predetermined period plus α minus the number of vaccines in stock.

The comparison order unit 16 then orders the shortage amounts calculated in step S94 through the communication Interface unit 30 (S95). The routine shown in Fig. 21 is thereafter finished.

Incidentally, the process shown in Fig. 21 may be performed not only by a medical facility but also by a local government or the like. In this case, based on the information of the designated medical facilities shown in Fig. 5, the calculation unit 15 reads the final schedules of all the preventive vaccination subjects to be vaccinated in all the designated medical facilities in the local government or the like. This is because the local government or the like can thereby obtain vaccines as own stock and distribute the vaccines to each medical facility.

By this configuration, in accordance with the vaccine scheduling device 1, the vaccine scheduling program and the computer-readable recording medium storing this program of the first embodiment, a final schedule is created by shifting the scheduled administration date of the vaccines to be administered after the fixed dates of the clinical history data to the future. Because of this, it is possible to create a final schedule by setting a prohibition period in appropriate manner for a designated person who has the measles, the rubella, the chicken pox or the like. Accordingly, it is possible to create a schedule taking into consideration the prohibition period of a vaccine based on the clinical history, and create a preventive vaccination schedule in an appropriate manner for a preventive vaccination subject.

Also, since a schedule is created for vaccinations received by a designated person based on information about applicability of simultaneous vaccination and the maximum number of simultaneous vaccinations of vaccines to be administered to the designated person, a schedule is created to perform separate vaccinations if simultaneous vaccination is not applicable, and a schedule is created to administer a plurality of vaccines at the same time if simultaneous vaccination is applicable. Accordingly, it is possible to create a preventive vaccination schedule in a more appropriate manner for a preventive vaccination subject.

Furthermore, it is possible to automate the ordering of vaccines, which is difficult to control the inventory thereof, and improve convenience at medical facilities by collecting the created final schedules of a plurality of persons, calculating the types and numbers of vaccines to be administered, comparing the calculated numbers with the stock and ordering the shortage amounts.

Next, a second embodiment of the present invention will be explained. While a vaccine scheduling device, a vaccine scheduling program and a computer-readable recording medium storing this program in accordance with the second embodiment are similar to those of the first embodiment, there are some different configuration and procedure therebetween. In what follows, the differences from the first embodiment will be explained.

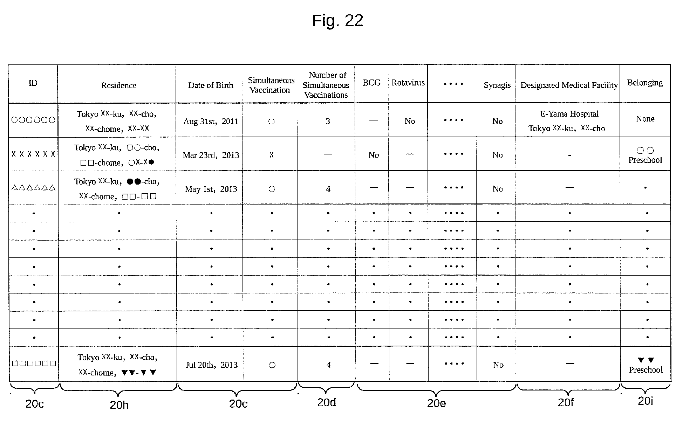

Fig. 22 is a view for showing the stored contents of the HDD 50 and the USB 60 in accordance with the second embodiment. As illustrated in Fig. 22, the HDD 50 and the USB 60 of the second embodiment store the information 20c of ID numbers in correspondence with the information 20c to 20f and 20h as shown in Fig. 5, and also in association with the information 20i about belonging.

Also, although not shown in the figures in the second embodiment, input boxes are provided in the display screens shown in Fig. 3 and Fig. 4 for inputting these information items. Users then input belonging groups to the input boxes so that the information 20c to 20f, 20h and 20i can be stored in association with each other in the HDD 50 and the USB 60.

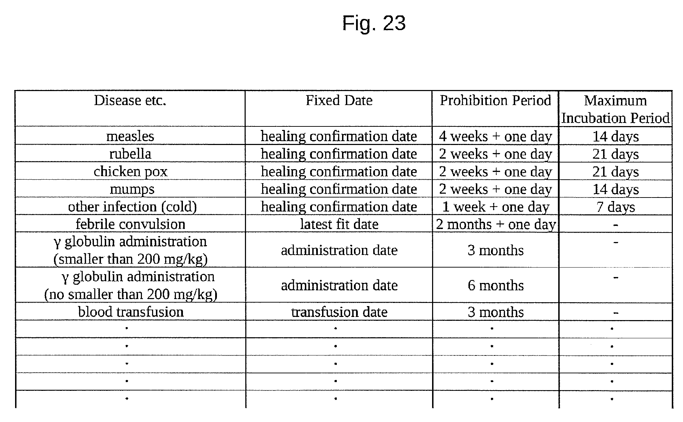

Fig. 23 is a view for showing the stored contents stored in a third storage area in accordance with the second embodiment. Like the example shown in Fig. 8, the third storage area (third storage means) is used to store prohibition periods in which vaccination is prohibited after the fixed dates of a plurality of diseases respectively. In addition to this, the maximum value of the incubation period of each disease is stored in the third storage area of the second embodiment. The incubation period is the time elapsed between infection with a pathogen and when symptoms are first apparent, for example, 14 days in the case of the measles. Incidentally, the incubation period is applicable also to viral diseases, and the information about an incubation period is not provided for febrile convulsion, administration of gamma globulin, blood transfusion and so forth.

Furthermore, in the case of the second embodiment, the clinical history data receiving unit 14 receives not only the clinical history data of a designated person but further receives the clinical history data of at least either of the family of the designated person (the family as used herein includes persons having the same residence) and a group to which the designated person belongs (kindergarten, nursery school, elementary school, middle school, high school, university, company, circle and so forth).

Because of this, the final schedule creation unit 13b performs processing based on the clinical history data which has been further received. Namely, the final schedule creation unit 13b reads the information about the prohibition period and the information about the maximum incubation period corresponding to the past diseases contained in the further received clinical history data from the third storage area. The final schedule creation unit 13b then calculates an infection development period by subtracting the number of days, which elapsed from the development confirmation date of a past disease indicated by the further received clinical history data to the current date, from the maximum incubation period of this disease. This infection development period is a period from when the designated person will develop a disease in future to when the prohibition period of this disease will elapse. Namely, in the case of the second embodiment, it is assumed that while the designated person has not developed a disease yet, the designated person is infected with a disease through the family of the designated person or the like.

Specifically, the final schedule creation unit 13b sets an added period by adding the prohibition period of a disease to an infection development period from the development confirmation date, and creates a final schedule by, in the case where the scheduled administration date of a vaccine(s) described in the provisional schedule created by the provisional schedule creation unit 13a falls in the period from the current time to the last day of the added period, shifting the scheduled administration date of a vaccine to be administered after the development confirmation date of the clinical history data to the future in accordance with the differential number of days between the release day when the added period ends and the earliest scheduled administration date of the vaccine(s) falling in the period.

This will be explained in detail. First, it is assumed that there is a person having developed the measles in the family or group of the designated person. In this case, the designated person can be infected. Since the maximum incubation period of the measles is 14 days, in the case where there is a person having developed the measles in the family or group 10 days before, the designated person may develop the measles within 4 days if the remaining 4 days (14 days - 10 days) has not elapsed.

In the case where there is a person having developed the measles in the family or group 10 based on the clinical history data, the final schedule creation unit 13b calculates the infection development period by subtracting the number of days elapsed between the current day and the development confirmation date of the measles from the maximum incubation period. Namely, the final schedule creation unit 13b calculates the maximum incubation period (the maximum value of the incubation period of the measles) - 10 days (the number of days elapsed between the current day and the development confirmation date of the measles) as 4 days, and determines 4 days as the infection development period.

Also, since the designated person may develop the measles at latest 4 days later, the designated person cannot receive preventive vaccination until 4 days + 4 weeks + 1 day elapses after the current day (i.e., the infection development period + the prohibition period of the measles = the added period). Accordingly, in the case where the scheduled administration date of a vaccine(s) described in the provisional schedule created by the provisional schedule creation unit 13a falls in the period 4 weeks + 5 days from the current time, the final schedule creation unit 13b adjusts the provisional schedule and create a final schedule.

Namely, in the case where the scheduled administration date of a vaccine(s) described in the provisional schedule created by the provisional schedule creation unit 13a falls in the period 4 weeks + 5 days from the current time, the final schedule creation unit 13b calculates the differential number of days between the release day when the added period ends and the earliest scheduled administration date of the vaccine(s) falling in the period. For example, if the earliest scheduled administration date is 2 weeks later, the differential number of days is calculated by performing an arithmetic operation of 4 weeks + 5 days + 1 day (1 day is the release day) - 2 weeks to obtain the differential number of days as 2 weeks + 6 days (i.e., 20 days).

The final schedule creation unit 13b then shifts the scheduled administration date of vaccines to be administered after the current time to future to create a final schedule. In doing so, while the scheduled administration date of vaccines to be administered after the current time can be shifted to future by 20 days, it is desirable to take the number of gap days into consideration in the same manner as in the first embodiment.

Incidentally, needless to say, the aforementioned process is not performed in the case where, even if there is a person in the family or group who developed the measles 20 days before, the maximum incubation period has already elapsed and it is determined that the designated person does not develop the measles. This is because the final schedule creation unit 13b determines that there is no scheduled administration date between the current time and the last day of the added period. Namely, the period between the current time and the last day of the added period is meant the period between the current time and the last day of the added period which is in future, and therefore the determination is not performed to the period between the current time and the last day of the added period which is in the past.

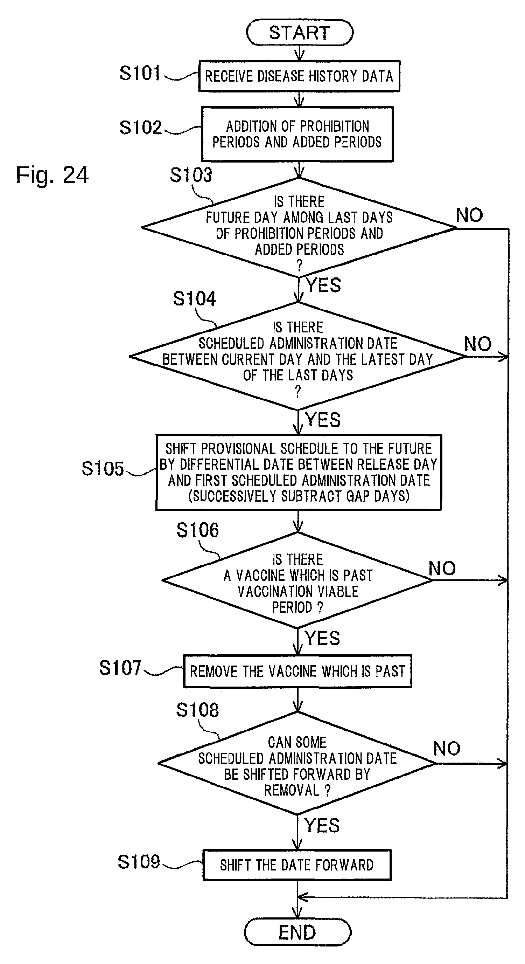

Fig. 24 is a flow chart for showing the final schedule creation process (S3) in accordance with the second embodiment in detail. As illustrated in Fig. 24, first, the clinical history data receiving unit 14 receives the clinical history data of the designated person, and further receives the clinical history data of at least either of the family of the designated person and a group to which the designated person belongs.

Next, the final schedule creation unit 13b adds the prohibition period of each of all the diseases of the clinical history data received in step S101 to the each disease (S102). Furthermore, with respect to each of all the diseases of the clinical history data of the family or group received in step S101, the final schedule creation unit 13b calculates an infection development period by subtracting the number of days, which elapsed from the development confirmation date to the current date, from the maximum incubation period of the each disease. With respect to the disease whose infection development period is negative, the following steps are not applied to the disease. With respect to the disease whose infection development period is positive, the prohibition period of the disease is added to the infection development period to calculate an added period. The final schedule creation unit 13b then adds the added period to the development confirmation date of the disease of each person belonging to the family or group (S102).

Next, the final schedule creation unit 13b determines whether or not there is a future day of the last days of the prohibition periods and added periods (S103). Meanwhile, the future in step S103 includes the current day.

If there is no future day (S103: NO), the process shown in Fig. 24 is finished. Namely, the provisional schedule created by the provisional schedule creation unit 13a is a final schedule as it is.

Conversely, if it is determined that there is a future day (S103: YES), the final schedule creation unit 13b determines whether or not there is a scheduled administration date between the current day and the latest day of the last days of the prohibition periods and added periods which are determined as in the future in step S103 (S104). In this process, the final schedule creation unit 13b determines whether or not a scheduled administration date therebetween with reference to the provisional schedule created by the provisional schedule creation unit 13a. Incidentally, the term "therebetween" includes the case where the current day and the latest day are the same day.

If it is determined that there is no scheduled administration date therebetween (S104: NO), the process shown in Fig. 24 is finished. Namely, the provisional schedule created by the provisional schedule creation unit 13a is a final schedule as it is.

If it is determined that there is a scheduled administration date therebetween (S104: YES), the final schedule creation unit 13b creates the final schedule creation unit by shifting the provisional schedule to the future in accordance with the differential date (for example, by the differential date) between the above release day (this release day is the last day of the prohibition period + one day, and the last day of the added period + one day) and the first (the closest future from the current time) scheduled administration date (S105).

Thereafter, the process in steps S106 to S109 is performed. This process is the same as the above process in steps S86 to S99 shown in Fig. 20, and therefore explanation is not repeated. Also, in step S105, it is desirable to take the number of gap days into consideration as described above.

As has been discussed above, in accordance with the vaccine scheduling device 1, the vaccine scheduling program and the computer-readable recording medium storing this program of the second embodiment, the similar advantages can be achieved as in the first embodiment.

Furthermore, in accordance with the second embodiment, a schedule for preventive vaccination of the designated person is created based on clinical history data by further receiving the clinical history data of at least either of the family of the designated person and a group to which the preventive vaccination subject belongs. For this reason, even for a designated person who has not yet the measles, the rubella, the chicken pox or the like, it is possible to create a schedule by taking the incubation period into consideration if development is possible in the future. Accordingly, it is possible to create a preventive vaccination schedule in a more appropriate manner for a preventive vaccination subject.

Next, a third embodiment of the present invention will be explained. While a vaccine scheduling device, a vaccine scheduling program and a computer-readable recording medium storing this program in accordance with the third embodiment are similar to those of the first embodiment, there are some different configuration and procedure therebetween. In what follows, the differences from the first embodiment will be explained.

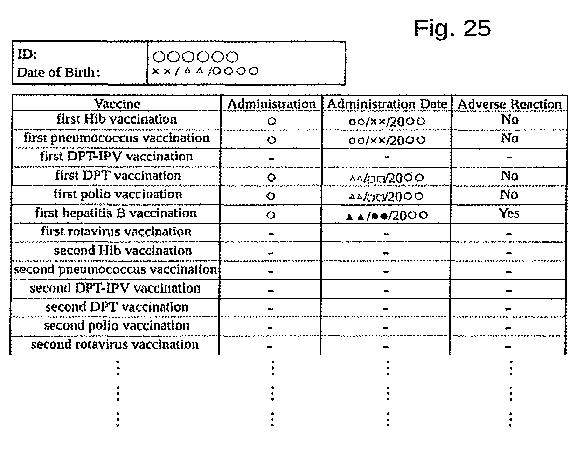

Fig. 25 is a view for showing the stored contents of the second storage area in accordance with the third embodiment. As illustrated in Fig. 25, in accordance with the third embodiment, the second storage area is used to store information about the ID number and birth date of each of a plurality of preventive vaccination subjects, information indicating that vaccination has been carried out ("○" in Fig. 7) and information indicating that vaccination has not been carried out yet (" -" in Fig. 7).

Still further, the second storage area (fourth storage means) is used to store information indicating whether or not an adverse effect occurs after administering a vaccine to the preventive vaccination subject. In this case, the adverse effect is, for example, occurrence of anaphylaxis or hives. When the adverse effect occurs, a doctor inputs the information about the occurrence to the vaccine scheduling device 1, and stores the information in the HDD 50 and USB 60. The information as shown in Fig. 25 is thereby formed.

Also, in accordance with the third embodiment, the schedule creation unit 13 creates a schedule with respect to vaccines which are not of the same type as vaccines with which adverse effects occur. Vaccines of the same type are, for example, hepatitis B vaccines for the first and second vaccinations, or vaccines having the same main ingredient (active ingredient). Accordingly, in the case where the first hepatitis B vaccine causes an adverse effect, the schedule creation unit 13 does not create a schedule for the second and third hepatitis B vaccine, and for other vaccines containing HBs antigen which is an active ingredient of the hepatitis B vaccine in order not to cause further adverse effect.

Incidentally, while the second storage area is used to store the information indicating whether or not an adverse effect occurs in accordance with the third embodiment, the fourth storage area may be used to store the information in association with the information about the names of vaccines.

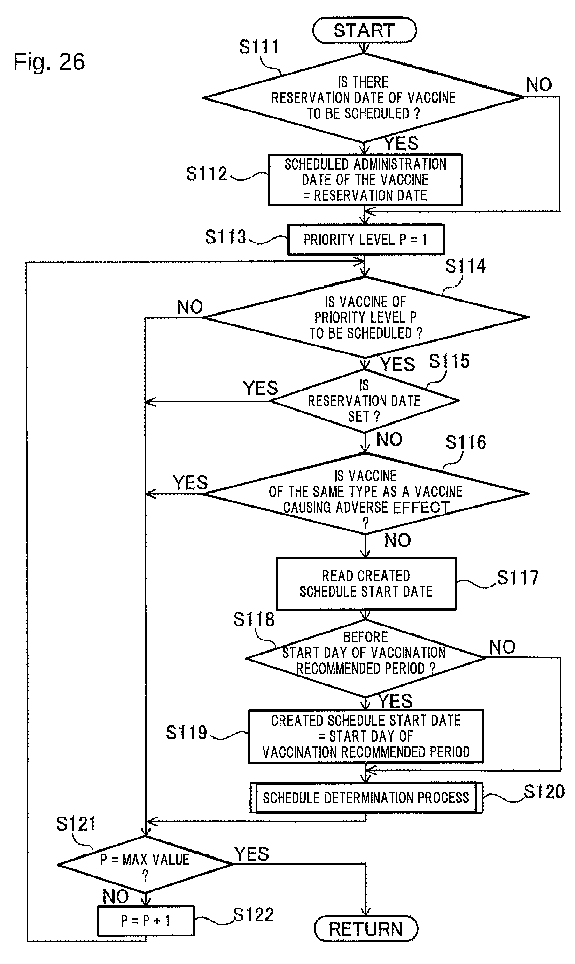

Fig. 26 is a flow chart for showing the provisional schedule creation process (S2) in accordance with the third embodiment in detail. The process in steps S111 to S 115 and S117 to S122 shown in Fig. 26 is the same as the process in steps S21 to S31 shown in Fig. 15, and therefore explanation is not repeated.

If it is determined that there is no reservation date for the vaccine of the priority level P (S115: NO), the provisional schedule creation unit 13a determines whether or not the vaccine of the priority level P is of the same type as a vaccine causing an adverse effect (S116). If it is determined that the vaccine of the priority level P is not of the same type, the process proceeds to step S117. Conversely, if it is determined that the vaccine of the priority level P is of the same type, the process proceeds to step S121 and then step S 122 in which the priority level P is incremented. Namely, the vaccine of the same type as a vaccine causing an adverse effect is not scheduled.

Incidentally, not shown in the figures and needless to say, the HDD 50 and the USB 60 store information about active ingredients of vaccines and association information indicating vaccines of the same type.

As has been discussed above, in accordance with the vaccine scheduling device 1, the vaccine scheduling program and the computer-readable recording medium storing this program of the third embodiment, the similar advantages can be achieved as in the first embodiment.

Furthermore, in accordance with the third embodiment, since a schedule (higher concept than the provisional schedule and the final schedule) is created based on whether or not an adverse effect of the designated person has occurred after administering a vaccine, it is possible to create a preventive vaccination schedule in a more appropriate manner for the preventive vaccination subject because, for example, when the designated person causes anaphylaxis after administering a particular vaccine, a vaccine of the same type as the particular vaccine is not scheduled.

Next, a fourth embodiment of the present invention will be explained. While a vaccine scheduling device, a vaccine scheduling program and a computer-readable recording medium storing this program in accordance with the fourth embodiment are similar to those of the first embodiment, there are some different configuration and procedure therebetween. In what follows, the differences from the first embodiment will be explained.

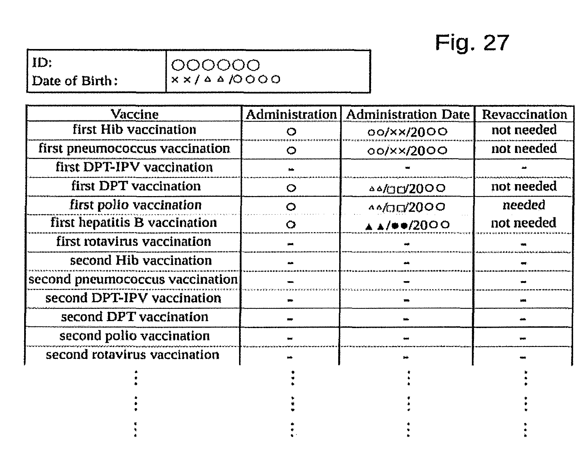

Fig. 27 is a view for showing the stored contents of the second storage area in accordance with the fourth embodiment. As illustrated in Fig. 27, in accordance with the third embodiment, the second storage area is used to store information about the ID number and birth date of each of a plurality of preventive vaccination subjects, information indicating that vaccination has been carried out ("○" in Fig. 7) and information indicating that vaccination has not been carried out yet ("-" in Fig. 7).

Furthermore, the second storage area (fifth storage means) is used to store information indicating, of the vaccines administered to the preventive vaccination subjects in the past, the vaccines needed to be administered again. Incidentally, there are vaccines which are less effective in some years (for example, the poliovirus vaccine administered to persons born in 1975 through 1977). If such years have been identified, doctors input the corresponding information for all the preventive vaccination subjects receiving the less effective vaccines, and store the information in the HDD 50 and the USB 60. The information as shown in Fig. 27 is thereby formed.

Meanwhile, in many cases, it requires several years or longer to confirm whether or not a vaccine is less effective. Accordingly, the information about revaccination shown in Fig. 27 is initialized as "not needed", and updated later as "needed" when it is confirmed that a vaccine is less effective. On the other hand, since the preventive vaccination subject receiving the less effective vaccines can be extracted with reference to the date of birth such as in the case of the poliovirus vaccine administered to persons born in 1975 through 1977, "needed" can be input generally to all the corresponding persons by entering "born in 1975 through 1977" and "poliovirus preventive vaccination" and so on.

Incidentally, while the second storage area is used to store the information indicating that revaccination is needed in accordance with the fourth embodiment, the fifth storage area may be used to store this information in association with the information about the names of vaccines.

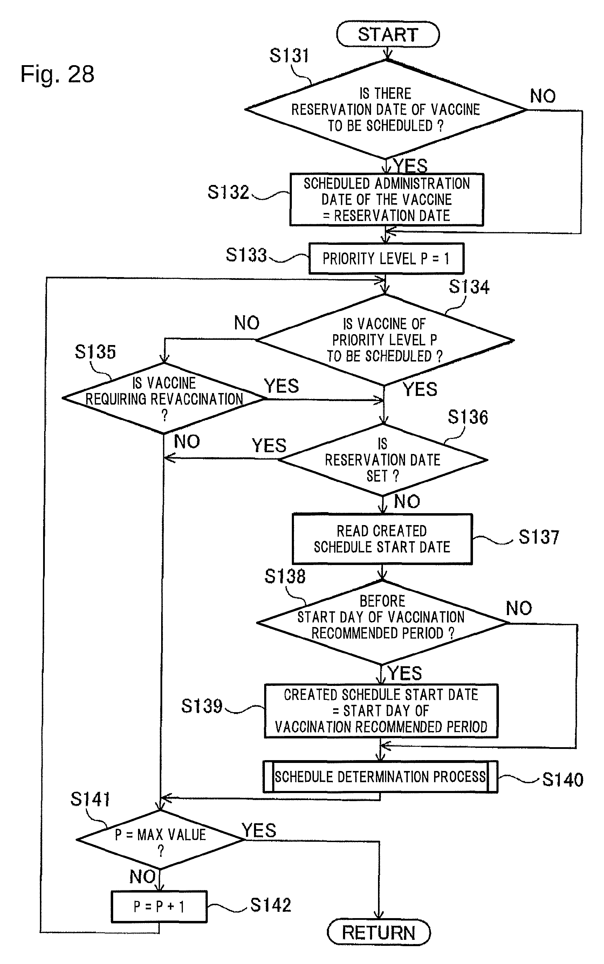

Fig. 28 is a flow chart for showing the provisional schedule creation process (S2) in accordance with the fourth embodiment in detail. The process in steps S 131 to S 134 and S 136 to S 142 shown in Fig. 28 is the same as the process in steps S21 to S31 shown in Fig. 15, and therefore explanation is not repeated.

If the vaccine of the priority level P is not to be scheduled (S134: NO), the provisional schedule creation unit 13a determines whether or not the vaccine of the priority level P requires revaccination (S 135). If it is determined that the priority level P does not require revaccination (S135: NO), the process proceeds to step S141.

Conversely, if it is determined that the priority level P requires revaccination (S135: YES), the process proceeds to step S136. If it is determined as "NO" in step S136, after processing in steps S137 to S139, the scheduled administration date is determined in step S140.

As has been discussed above, in accordance with the vaccine scheduling device 1, the vaccine scheduling program and the computer-readable recording medium storing this program of the fourth embodiment, the similar advantages can be achieved as in the first embodiment.

Furthermore, in accordance with the fourth embodiment, since a schedule (higher concept than the provisional schedule and the final schedule) is created with a vaccine which is administered to the designated person in the past and requires revaccination, it is possible to create a preventive vaccination schedule in a more appropriate manner for the preventive vaccination subject because, for example, if vaccinated in a year in which a vaccine was less effective, a schedule is created in order that this vaccine is to be administered again.

The image processing apparatus have been explained based on the embodiments in accordance with the present invention. However, it is not intended to limit the present invention to the precise form described, and obviously many modifications and variations are possible without departing from the scope of the invention.

For example, while the vaccine scheduling device 1 in accordance with the first embodiment is implemented with one personal computer, the present invention is not limited to this, but applicable to a vaccine scheduling system implemented with a plurality of computers which are connected through a network. Furthermore, the vaccine scheduling device 1 in accordance with the first embodiment may be implemented with a mobile terminal such as a tablet, a mobile device in place of a personal computer. At least one of a plurality of computers connected through a network may be a mobile terminal.

Also, in accordance with the first embodiment, the program for implementing the vaccine scheduling device 1 may be stored not only in the ROM 10a, the HDD 50 and the USB 60, but also in a CD-ROM, a CD-R or any other type of recording medium.

Furthermore, while the vaccine scheduling device 1 of the second embodiment reads the clinical history data of the family of a preventive vaccination subject and a group to which the preventive vaccination subject belongs, as this clinical history data, information that can be read as much as possible or only the clinical history data of the family may be read. Also, the clinical history data to be read may be only for one person, but the number of persons is not limited.

Incidentally, in accordance with the third and fourth embodiments, the schedule creation unit 13 does not schedule a vaccine which is of the same type as a vaccine with which an adverse effect occurs, and does schedule a vaccine which requires revaccination even if this vaccine has been administered. However, in place of the schedule creation unit 13, the determination unit 12 may exclude, from scheduling, a vaccine which is of the same type as a vaccine with which an adverse effect occurs, and include, as a scheduling object, a vaccine which requires revaccination even if this vaccine has been administered. In other words, the determination unit 12 can determine whether or not a vaccine is of the same type as a vaccine with which an adverse effect occurs, and whether or not a vaccine requires revaccination.

Also, the vaccine scheduling device 1 may obtain all or part of the information, which is stored in the HDD 50 and the USB 60, through the communication Interface unit 30. In this case, the storage areas or the like are provided in a computer which is connected through a network.

### [REFERENCE SIGNS LIST]

- 1: : vaccine scheduling device
- 2: : keyboard
- 3: : mouse
- 10: : CPU
- 10a: : ROM
- 10b: : RAM
- 11: : designation unit (designation means)
- 12: : determination unit (determination means)
- 13: : schedule creation unit (schedule creation means)
- 13a: : provisional schedule creation unit (provisional schedule creation means)
- 13b: : final schedule creation unit (final schedule creation means)
- 14: : clinical history data receiving unit (clinical history data receiving means)
- 15: : calculation unit (calculation means)
- 16: : comparison order unit (comparison order means)
- 20: : display
- 30: : communication Interface unit
- 50: : HDD (first through fifth storage means)
- 60: : USB (first through fifth storage means)

## Claims

1. A vaccine scheduling device structured to create a schedule of preventive vaccination, comprising:
a first storing means which stores information about a plurality of vaccines to be administered, and stores, in association with the information about the plurality of vaccines respectively, the information about priority levels of the vaccines, and information about the interval period to be inserted when administering, after each vaccine is administered, another vaccine which is different from the each vaccine;
a second storing means which stores information about vaccines which have been administered to a plurality of preventive vaccination subjects respectively;
a designation means structured to designate one of the plurality of preventive vaccination subjects as a designated person;
a determination means structured to select vaccines one by one in the descending order of the priority levels based on the information about the priority levels stored in association with the information of the vaccines stored in the first storing means, and successively determine whether or not the selected vaccine has been administered to the designated person based on the information stored in the second storing means;
a schedule creation means structured to create a schedule with the vaccines successively determined by the determination means as not having administered to the designated person;
a third storing means which stores, for each of a plurality of diseases, information about a prohibition period in which administration of the each vaccine is prohibited from a fixed date of the each disease; and
a clinical history data receiving means which receives clinical history data which includes past diseases of the designated person and fixed dates thereof,
the schedule creation means comprising:
a provisional schedule creation means which successively selects, one by one in the descending order of the priority levels, the vaccines which are successively determined by the determination means as not having administered to the designated person, sets a candidate administration date of the selected vaccine, resets the candidate administration date to the earliest date after the interval period of a vaccine which is administered just before the selected vaccine, and determines the reset candidate administration date as the scheduled administration date of the selected vaccine; and
a final schedule creation means which reads, from a third storing means, the prohibition period corresponding to a past disease of the clinical history data of the designated person received by the clinical history data receiving means, sets the prohibition period from the fixed date of the past disease of the clinical history data, and in the case where the scheduled administration date of a vaccine(s) is between the present time and the last day of the prohibition period in accordance with a provisional schedule created by the provisional schedule creation means, creates a final schedule by shifting to the future, in accordance with the differential number of days between the earliest scheduled administration date of the vaccine(s) and the release day when the prohibition period ends, the scheduled administration dates of vaccines to be administered after the fixed date of the past disease of the clinical history data.

2. The vaccine scheduling device of Claim 1 wherein
the clinical history data receiving means further receives the clinical history data of at least either of the family of the designated person and a group to which the designated person belongs, wherein
the further received clinical history data includes information about a past disease, the development confirmation date of the past disease, and the fixed date of the past disease, wherein
the third storing means stores, with respect to each of a plurality of diseases, information about a prohibition period in which vaccination is prohibited after the fixed dates of the each disease, and information about the maximum value of an incubation period, and wherein
the final schedule creation means reads the information about the prohibition period and the information about the maximum incubation period corresponding to the past diseases contained in the further received clinical history data from the third storage means, calculates an infection development period by subtracting the number of days, which elapsed from the development confirmation date of a past disease indicated by the further received clinical history data to the current date, from the maximum incubation period of this disease, sets an added period by adding the prohibition period of the disease to an infection development period, and creates a final schedule by, in the case where the scheduled administration date of a vaccine(s) described in the provisional schedule created by the provisional schedule creation means falls in the period from the current time to the last day of the added period, shifting the scheduled administration date of a vaccine(s) to be administered after the current time to the future in accordance with the differential number of days between the release day when the added period ends and the earliest scheduled administration date of the vaccine(s) falling in the period.

3. The vaccine scheduling device of either of Claim 2 and Claim 3 wherein the provisional schedule creation means determines whether or not the candidate administration date set to the earliest date is within a period in which a public expense is paid to the selected vaccine, determines that the selected vaccine is not administered if the earliest date is out of the period in which the public expense is paid and does not schedule the selected vaccine to a scheduled administration date.

4. The vaccine scheduling device of any one of Claim 1 through Claim 3 further comprising a fourth storing means which stores information about whether or not an adverse reaction occurs after administering a vaccine to the designated person, and wherein
the schedule creation means is structured to schedule, of the vaccines successively determined by the determination means as not having administered to the designated person, a vaccine which is not of the same type as a vaccine with which an adverse reaction occurs.

5. The vaccine scheduling device of any one of Claim 1 through Claim 4 further comprising a fifth storing means which stores information about a vaccine which is administered to the designated person in the past and requires revaccination, and wherein
the schedule creation means is structured to schedule the vaccines successively determined by the determination means as not having administered to the designated person, and the vaccine requiring revaccination stored in the fifth storing means.

6. The vaccine scheduling device of any one of Claim 1 through Claim 5 further comprising:
a calculation means which collects final schedules for a plurality of persons created by the final schedule creation means, and calculates the types and numbers of vaccines to be administered in a predetermined period; and
a comparison order means which compares the types and numbers of vaccines to be administered in the predetermined period calculated by the calculation means with the types and numbers of vaccines in stock, and ordering shortage amounts.

7. A vaccine scheduling program which causes a vaccine scheduling device structured to create a schedule of preventive vaccination and comprising a first storing means which stores information about a plurality of vaccines to be administered, and stores, in association with the information about the plurality of vaccines respectively, the information about priority levels of the vaccines, and information about the interval period to be inserted when administering, after each vaccine is administered, another vaccine which is different from the each vaccine; a second storing means which stores information about vaccines which have been administered to a plurality of preventive vaccination subjects respectively; and a third storing means which stores, for each of a plurality of diseases, information about a prohibition period in which administration of the each vaccine is prohibited from a fixed date of the each disease, to perform:
a designation step of designating one of the plurality of preventive vaccination subjects as a designated person;
a determination step of selecting vaccines one by one in the descending order of the priority levels based on the information about the priority levels stored in association with the information of the vaccines stored in the first storing means, and successively determining whether or not the selected vaccine has been administered to the designated person based on the information stored in the second storing means;
a schedule creation step of creating a schedule with the vaccines successively determined by the determination step as not having administered to the designated person; and
a clinical history data receiving step of receiving clinical history data which includes past diseases of the designated person and fixed dates thereof,
the schedule creating step comprising:
a provisional schedule creation step of successively selecting, one by one in the descending order of the priority levels, the vaccines which are successively determined in the determination step as not having administered to the designated person, setting a candidate administration date of the selected vaccine, resetting the candidate administration date to the earliest date after the interval period of a vaccine which is administered just before the selected vaccine, and determining the reset candidate administration date as the scheduled administration date of the selected vaccine; and
a final schedule creation step of reading, from a third storing means, the prohibition period corresponding to a past disease of the clinical history data of the designated person received by the clinical history data receiving step, setting the prohibition period from the fixed date of the past disease of the clinical history data, and in the case where the scheduled administration date of a vaccine(s) is between the present time and the last day of the prohibition period in accordance with a provisional schedule created by the provisional schedule creation step, creating a final schedule by shifting to the future, in accordance with the differential number of days between the earliest scheduled administration date of the vaccine(s) and the release day when the prohibition period ends, the scheduled administration dates of vaccines to be administered after the fixed date of the past disease of the clinical history data.

8. A computer-readable recording medium storing the vaccine scheduling program as recited in Claim 7.
